(19) **Europäisches Patentamt**
**European Patent Office**
**Office européen des brevets**

(11) **EP 3 093 001 A1**

(12) **EUROPEAN PATENT APPLICATION**

(43) Date of publication:
**16.11.2016 Bulletin 2016/46**

(51) Int Cl.:
*A61K 8/04* *(2006.01)*     *A61K 8/894* *(2006.01)*
*A61K 8/31* *(2006.01)*     *A61K 8/37* *(2006.01)*
*A61Q 1/06* *(2006.01)*     *A61Q 1/02* *(2006.01)*
*A61Q 17/04* *(2006.01)*     *A61Q 19/10* *(2006.01)*
*A61Q 5/06* *(2006.01)*     *A61Q 5/12* *(2006.01)*
*A61Q 5/00* *(2006.01)*     *A61Q 19/00* *(2006.01)*
*A61Q 5/02* *(2006.01)*     *A61Q 15/00* *(2006.01)*

(21) Application number: **16171079.3**

(22) Date of filing: **26.09.2008**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **26.09.2007 US 975318 P**

(62) Document number(s) of the earlier application(s) in accordance with Art. 76 EPC:
**08833137.6 / 2 194 954**

(71) Applicant: **Dow Corning Corporation
Midland, Michigan 48686-0994 (US)**

(72) Inventors:
  • **KENNAN, John Joseph
    48640 Midland (US)**

  • **VAN REETH, Isabelle
    Pudong, Shanghai 201206 (CN)**
  • **MESSNER, Kathryn Elizabeth
    48640 Midland (US)**
  • **GARAUD, Jean-Luc
    59560 Comines (FR)**

(74) Representative: **f & e patent
    Fleischer, Engels & Partner mbB, Patentanwälte
    Braunsberger Feld 29
    51429 Bergisch Gladbach (DE)**

Remarks:
This application was filed on 24-05-2016 as a divisional application to the application mentioned under INID code 62.

(54) **PERSONAL CARE COMPOSITIONS CONTAINING SILICONE-ORGANIC GELS FROM POLYALKYLOXYLENE CROSSLINKED SILICONE ELASTOMERS**

(57) Silicone organic gels and gel paste compositions are disclosed containing a silicone organic elastomer in a carrier fluid. The silicone organic elastomer is a reaction product of a linear or branched organohydrogensiloxane, an $\alpha, \omega$ - unsaturated polyoxyalkylene, and a hydrosilylation catalyst. The silicone organic elastomer is particularly useful to gel organic carrier fluids. The gels and gels pastes also provide enhanced compatibility with many personal and health care actives.

EP 3 093 001 A1

**Description**

Cross Reference to Related Applications

This application claims priority to US 60/975,318, filed on September 26, 2007.

Technical Field

This disclosure relates to personal care compositions containing silicone organic gels or gel paste compositions. The gel or gel paste compositions are formed from a silicone organic elastomer in a carrier fluid. The silicone organic elastomer is a reaction product of a linear or branched organohydrogensiloxane, an $\alpha, \omega$ - unsaturated polyoxyalkylene, and a hydrosilylation catalyst.

Background

Silicone elastomer gels have been used extensively to enhance the aesthetics of personal care formulations by providing a unique sensory profile upon application. Most silicone elastomer gels are obtained by a crosslinking hydrosilylation reaction of an SiH polysiloxane with another polysiloxane containing an unsaturated hydrocarbon substituent, such as a vinyl functional polysiloxane, or by crosslinking an SiH polysiloxane with a hydrocarbon diene. The silicone elastomers may be formed in the presence of a carrier fluid, such as a volatile silicone, resulting in a gelled composition. Alternatively, the silicone elastomer may be formed at higher solids content, subsequently sheared and admixed with a carrier fluid to also create gels or paste like compositions. Representative examples of such silicone elastomers are taught in U.S. Patent 5,880,210, and U.S. 5,760,116.

While silicone elastomers have provided significant advances for improving personal care formulation, they possess several shortcomings that have limited their use. For example, silicone elastomers having mostly dimethyl siloxane content are less effective for gelling organic based solvents and carrier fluids. Silicone elastomer gel compositions having high dimethyl siloxane also have limited compatibility with many personal care ingredients. For example, the widely used sunscreen agent, octyl methoxycinnamate, has limited solubility in many of these silicone elastomer gels. Another problem is the reduction of viscosity of the silicone elastomer gel in the presence of such incompatible components. Thus, there is a need to identify silicone elastomers that can gel organic solvents. Furthermore, there is a need to identify silicone elastomer gels having improved compatibilities with many personal care ingredients, while maintaining the aesthetics associated with silicone organic elastomer gels. To this end, there have been many attempts to improve compatibilities of silicone elastomers with various personal care ingredients wherein alkyls, polyether, amines or other organofunctional groups have been grafted onto the silicone organic elastomer backbone. Representative of such organofunctional silicone elastomers are taught in US 5,811,487 , US 5,880,210 , US 6,200,581, US 5,236,986, US 6,331,604, US 6,262,170, US 6,531,540, and US 6,365,670.

However, there is still a need to improve the compatibility of silicone elastomer based gels, and in particular, with organic based volatile fluids and personal care ingredients. Such improved compatibility should not sacrifice sensory aesthetic profiles. Furthermore, the gelling or thickening efficiency of the silicone elastomer in a carrier fluid should be maintained or improved.

The present inventors have discovered silicone organic elastomers based on certain polyoxyalkylene crosslinkers provide gelled compositions of carrier fluids efficiently. The resulting gelled compositions also possess additional benefits, such as improved compatibilities with many common personal care ingredients, while maintaining sensory aesthetics and therefore are useful in a variety of personal care and healthcare formulations.

Summary

This disclosure relates to personal or healthcare compositions comprising a silicone organic elastomer gel derived from;

i) a silicone organic elastomer comprising a reaction product of;

A) an organohydrogensiloxane comprising siloxy units of average formula

$$(R^1{}_3SiO_{0.5})_v(R^2{}_2SiO)_x(R^2HSiO)_y$$

wherein $R^1$ is hydrogen or $R^2$,

$R^2$ is a monovalent hydrocarbyl,

$$v \geq 2, x \geq 0, y \geq 2,$$

B) a polyoxyalkylene comprising the average formula

$$R^3O-[(C_2H_4O)_c \ (C_3H_6O)_d \ (C_4H_8O)_e]-R^3$$

wherein

$R^3$ is a monovalent unsaturated aliphatic hydrocarbon group containing 2 to 12 carbon atoms,
c is from 0 to 100,
d is from 0 to 100,
e is from 0 to 100,
with a proviso the ratio of (d + e)/(c + d + e) is greater than 0.5,

C) a hydrosilylation catalyst, and
D) an optional organic compound having one terminal unsaturated aliphatic hydrocarbon group,

ii) a carrier fluid, and

E) a personal or healthcare active.

Detailed Description

The personal or healthcare compositions of the present disclosure comprise a silicone organic elastomer gel. The silicone organic gel and gel paste compositions of the present disclosure contain i) a silicone organic elastomer (from the reaction of components A), B), C) and optionally D)), ii) a carrier fluid, and E) a personal or health care active.

*(i) The Silicone Organic Elastomer*

The silicone organic elastomers of the present disclosure are obtainable as hydrosilylation reaction products of an organohydrogensiloxane, an $\alpha$, $\omega$ - unsaturated polyoxyalkylene, and a hydrosilylation catalyst, components A), B), and C) respectively. The term "hydrosilylation" means the addition of an organosilicon compound containing silicon-bonded hydrogen, (such as component A) to a compound containing aliphatic unsaturation (such as component B), in the presence of a catalyst (such as component C). Hydrosilylation reactions are known in the art, and any such known methods or techniques may be used to effect the hydrosilylation reaction of components A), B), and C) to prepare the silicone organic elastomers as component i) of the present disclosure.

The silicone organic elastomer may also contain pendant, non-crosslinking moieties, independently selected from hydrocarbon groups containing 2 - 30 carbons, polyoxyalkylene groups, and mixtures thereof. Such pendant groups result from the optional addition of component D') a hydrocarbon containing 2-30 carbons having one terminal unsaturated aliphatic group, and/or component D") a polyoxyalkylene having one terminal unsaturated aliphatic group to the silicone organic elastomer via a hydrosilylation reaction.

The hydrosilylation reaction to prepare the silicone organic elastomer may be conducted in the presence of a solvent, and the solvent subsequently removed by known techniques. Alternatively, the hydrosilylation may be conducted in a solvent, where the solvent is the same as the carrier fluid described as component ii).

*A) The Organohydrogensiloxane*

Component A) of the present invention is a linear or branched organohydrogensiloxane having an average, per molecule, of at least two SiH units. As used herein, an organohydrogensiloxane is any organopolysiloxane containing a silicon-bonded hydrogen atom (SiH). Organopolysiloxanes are polymers containing siloxy units independently selected from $(RSiO_{0.5})$, $(R_2SiO)$, $(RSiO_{1.5})$, or $(SiO_2)$ siloxy units, where R may be any organic group. When R is a methyl group in the $(R_3SiO_{0.5})$, $(R_2SiO)$, $(RSiO_{1.5})$, or $(SiO_2)$ siloxy units of an organopolysiloxane, the siloxy units are commonly referred to as M, D, T, and Q units respectively. These siloxy units can be combined in various manners to form cyclic, linear, or branched structures. The chemical and physical properties of the resulting polymeric structures can vary. For example organopolysiloxanes can be volatile or low viscosity fluids, high viscosity fluids/gums, elastomers or rubbers, and resins.

Organohydrogensiloxanes are organopolysiloxanes having at least one SiH containing siloxy unit, that is at least one siloxy unit in the organopolysiloxane has the formula $(R_2HSiO_{0.5})$, $(RHSiO)$, or $(HSiO_{1.5})$. Thus, the organohydrogensiloxanes useful in the present invention may comprise any number of $(R_3SiO_{0.5})$, $(R_2SiO)$, $(RSiO_{1.5})$, $(R_2HSiO_{0.5})$, $(RHSiO)$, $(HSiO_{1.5})$ or $(SiO_2)$ siloxy units, providing there are on average at least two SiH siloxy units in the molecule, and the organohydrogensiloxane is linear or branched. As used herein, "linear or branched" organohydrogensiloxane excludes cyclic organohydrogensiloxane structures. Component (A) can be a single linear or branched organohydrogensiloxane or a combination comprising two or more linear or branched organohydrogensiloxanes that differ in at least one of the following properties; structure, viscosity, average molecular weight, siloxane units, and sequence.

The organohydrogensiloxane may have the average formula;

$$(R^1{}_3SiO_{0.5}MR^2{}_2SiOMR^2HSiO)_y$$

wherein

$R^1$ is hydrogen or $R^2$,
$R^2$ is a monovalent hydrocarbyl,

$v \geq 2$,
$x \geq 0$, alternatively x = 1 to 500, alternatively x = 1 to 200,
$y \geq 2$, alternatively y = 2 to 200, alternatively y = 2 to 100.

$R^2$ may be a substituted or unsubstituted aliphatic or aromatic hydrocarbyl. Monovalent unsubstituted aliphatic hydrocarbyls are exemplified by, but not limited to alkyl groups such as methyl, ethyl, propyl, pentyl, octyl, undecyl, and octadecyl and cycloalkyl groups such as cyclohexyl. Monovalent substituted aliphatic hydrocarbyls are exemplified by, but not limited to halogenated alkyl groups such as chloromethyl, 3-chloropropyl, and 3,3,3-trifluoropropyl. The aromatic hydrocarbon group is exemplified by, but not limited to, phenyl, tolyl, xylyl, benzyl, styryl, and 2-phenylethyl.

In one embodiment, the organohydrogensiloxane may contain additional siloxy units and have the average formula

$$(R^1{}_3SiO_{0.5})_v(R^2{}_2SiO)_x(R^2HSiO)_y(R^2SiO_{1.5})_z,$$

$$(R^1{}_3SiO_{0.5})_v(R^2{}_2SiOMR^2HSiO)_y(SiO_2)_w,$$

$$(R^1{}_3SiO_{0.5})_v(R^2{}_2SiO)_x(R^2HSiO)_y(SiO_2)_w(R^2SiO_{1.5})_2$$

or any mixture thereof,
where

$R^1$ is hydrogen or $R^2$,
$R^2$ is a monovalent hydrocarbyl,
and $v \geq 2$, $w \geq 0$, $x \geq 0$, $y \geq 2$, and z is $\geq 0$.

In another embodiment, the organohydrogensiloxane is selected from a dimethyl, methyl-hydrogen polysiloxane having the average formula;

$$(CH_3)_3SiO[(CH_3)_2SiO]_x[(CH_3)HSiO]_ySi(CH_3)_3$$

where $x \geq 0$, alternatively, x = 1 to 500, alternatively x = 1 to 200,
and $y \geq 2$, alternatively, y = 2 to 200, alternatively y = 2 to 100.

Methods for preparing organohydrogensiloxanes are well known, and many are sold commercially.

*B) The Polyoxyalkylene*

Component B) is a polyoxyalkylene having an average formula

$$R^3O-[(C_2H_4O)_c(C_3H_6O)_d(C_4H_8O)_e]-R^3$$

wherein

$R^3$ is a monovalent unsaturated aliphatic hydrocarbon group containing 2 to 12 carbon atoms,
c is from 0 to 100,
d is from 0 to 100,
e is from 0 to 100,
with a proviso the ratio of (d + e)/(c + d + e) is greater than 0.5,
alternatively greater than 0.8,
or alternatively greater than 0.95.

The polyoxyalkylene useful as component B) can be any polyoxyalkylene that is terminated at each molecular chain end (i.e. alpha and omega positions) with a unsaturated aliphatic hydrocarbon group containing 2 to 12 carbon atoms. The polyoxyalkylene may result from the polymerization of ethylene oxide, propylene oxide, butylene oxide, 1,2-epoxyhexane, 1, 2-epoxyoctane, cyclic epoxides such as cyclohexene oxide or exo-2,3-epoxynorborane. The polyoxyalkylene group may comprise oxyethylene units ($C_2H_4O$), oxypropylene units ($C_3H_6O$), oxybutylene units ($C_4H_8O$), or mixtures thereof. Typically, the polyoxyalkylene group comprises a majority of oxypropylene or oxybutylene units, as defined on a molar basis and indicated in the above formula by the c, d, and e subscripts. The unsaturated aliphatic hydrocarbon group can be an alkenyl or alkynyl group. Representative, non- limiting examples of the alkenyl groups are shown by the following structures; $H_2C=CH-$, $H_2C=CHCH_2-$, $H_2C=C(CH_3)CH_2-$, $H_2C=CHCH_2CH_2-$, $H_2C=CHCH_2CH_2CH_2-$, and $H_2C=CHCH_2CH_2CH_2CH_2-$. Representative, non- limiting examples of alkynyl groups are shown by the following structures; $HC{\equiv}C-$, $HC{\equiv}CCH_2-$, $HC{\equiv}CC(CH_3)-$, $HC{\equiv}CC(CH_3)_2-$ , $HC{\equiv}CC(CH_3)_2CH_2-$.
Polyoxyalkylenes having an unsaturated aliphatic hydrocarbon group at each molecular terminal are known in the art, and many are commercially available. Representative, non-limiting examples of polyoxyalkylenes having an unsaturated aliphatic hydrocarbyl at each molecular terminal include;

$$H_2C=CHCH_2O[C_3H_6]_dCH_2CH=CH_2$$

$$H_2C=C(CH_3)CH_2O[C_3H_6]_dCH_2C(CH_3)=CH_2$$

$$HC{\equiv}CCH_2O[C_3H_6O]_dCH_2C{\equiv}CH$$

$$HC{\equiv}CC(CH_3)_2O[C_3H_6O]_dC(CH_3)_2C{\equiv}CH$$

where d is as defined above.
Polyoxyalkylenes having an unsaturated aliphatic hydrocarbon group at each molecular terminal are commercially available from NOF (Nippon Oil and Fat, Tokyo, Japan) and Clariant Corp. (Charlottesville, NC).
The amounts of components A) and B) used in the hydrosilylation reaction may vary. Typically, the molar ratio of the SiH units of component A) to the aliphatic unsaturated groups of component B) ranges from 10/1 to 1/10, alternatively from 5/1 to 1/5, or alternatively from 2/1 to 1/2.

*(C) The Hydrosilylation Catalyst*

Component (C) comprises any catalyst typically employed for hydrosilylation reactions. It is preferred to use platinum group metal-containing catalysts. By platinum group it is meant ruthenium, rhodium, palladium, osmium, iridium and platinum and complexes thereof. Platinum group metal-containing catalysts useful in preparing the compositions of the present invention are the platinum complexes prepared as described by Willing, U. S. Pat. No. 3,419,593, and Brown et al, U. S. Pat. No. 5,175,325, each of which is hereby incorporated by reference to show such complexes and their preparation. Other examples of useful platinum group metal-containing catalysts can be found in Lee et al., U. S. Pat. No. 3,989,668; Chang et al., U. S. Pat. No. 5,036,117; Ashby, U. S. Pat. No. 3,159,601; Lamoreaux, U. S. Pat. No. 3,220,972; Chalk et al., U. S. Pat. No. 3,296,291; Modic, U. S. Pat. No. 3,516,946; Karstedt, U. S. Pat. No. 3,814,730; and Chandra et al., U. S. Pat. No. 3,928,629 all of which are hereby incorporated by reference to show useful platinum group metal-containing catalysts and methods for their preparation. The platinum group-containing catalyst can be platinum group metal, platinum group metal deposited on a carrier such as silica gel or powdered charcoal, or a compound or complex of a platinum group metal. Preferred platinum-containing catalysts include chloroplatinic acid, either in hexahydrate form or anhydrous form, and or a platinum-containing catalyst which is obtained by a method comprising reacting chloroplatinic acid with an aliphatically unsaturated organosilicon compound such as divinyltetramethyldisiloxane, or alkene-platinum-silyl complexes as described in U.S. Patent Application No. 10/017229, filed December 7, 2001, such as $(COD)Pt(SiMeCl_2)_2$, where COD is 1,5-cyclooctadiene and Me is methyl. These alkene-platinum-silyl complexes may be prepared, for example by mixing 0.015 mole $(COD)PtCl_2$ with 0.045 mole COD and 0.0612 moles $HMeSiCl_2$.

The appropriate amount of the catalyst will depend upon the particular catalyst used. The platinum catalyst should be present in an amount sufficient to provide at least 2 parts per million (ppm), alternatively 4 to 200 ppm of platinum based on total weight percent solids (all non-solvent ingredients) in the composition. Typically, the platinum is present in an amount sufficient to provide 4 to 150 weight ppm of platinum on the same basis. The catalyst may be added as a single species or as a mixture of two or more different species.

*D) Optional components containing one terminal unsaturated aliphatic hydrocarbon group*

The silicone organic elastomer may also contain pendant, non-crosslinking moieties, independently selected from hydrocarbon groups containing 2 - 30 carbons, polyoxyalkylene groups, and mixtures thereof. These groups are formed on the silicone organic elastomer via a hydrosilylation reaction by the addition of component D) an organic compound having one terminal unsaturated aliphatic hydrocarbon group. Component D) may be selected from D') a hydrocarbon containing 6-30 carbons having one terminal unsaturated aliphatic hydrocarbon group, and/or component D") a polyoxyalkylene having one terminal unsaturated aliphatic group.

The addition of component D) can alter the resulting chemical and physical properties of the silicone organic elastomer. For example, selecting D' will result in the addition of hydrocarbon groups to the silicone organic elastomer, thus adding more hydrophobic character to the silicone organic elastomer. Conversely, selecting a polyoxyalkylene having a majority of ethylene oxide units will result in a silicone organic elastomer having increased hydrophilicity, which can subsequently incorporate water or hydrophilic components with the silicone organic elastomer to form dispersions or pastes.

The unsaturated aliphatic hydrocarbon group in D' or D" can be an alkenyl or alkynyl group. Representative, non- limiting examples of the alkenyl groups are shown by the following structures; $H_2C=CH-$, $H_2C=CHCH_2-$, $H_2C=C(CH_3)CH_2-$, $H_2C=CHCH_2CH_2-$, $H_2C=CHCH_2CH_2CH_2-$, and $H_2C=CHCH_2CH_2CH_2CH_2-$. Representative, non- limiting examples of alkynyl groups are shown by the following structures; $HC≡C-$, $HC≡CCH_2-$, $HC≡CC(CH_3)-$, $HC≡CC(CH_3)_2-$, and $HC≡CC(CH_3)_2CH_2-$.

Component D'), the hydrocarbon containing 6-30 carbons having one terminal unsaturated aliphatic group, may be selected from alpha olefins such as 1-hexene, 1-octene, 1-decene, 1- undecene, 1-decadecene, and similar homologs. Component D') may also be selected from aryl containing hydrocarbons such as alpha methyl styrene.

Component D") may be selected from those polyoxyalkylenes having the average

formula        $R^3O-[(C_2H_4O)_{c'}(C_3H_6O)_{d'}(C_4H_8O)_e]-R^4$

where $R^3$ is a monovalent unsaturated aliphatic hydrocarbon group containing 2 to 12 carbon atoms,
c' is from 0 to 100, d' is from 0 to 100, e is from 0 to 100, providing the sum of c', d', and e is > 0.

$R^4$ is hydrogen, an acyl group, or a monovalent hydrocarbon group containing 1 to 8 carbons. Representative, non-limiting examples of polyoxyalkylenes, useful as component D") include;

$H_2C=CHCH_2O(C_2H_4O)_{c'}H$
$H_2C=CHCH_2O(C_2H_4O)_{c'}CH_3$
$H_2C=CHCH_2O(C_2H_4O)_{c'}C(O)CH_3$
$H_2C=CHCH_2O(C_2H_4O)_{c'}(C_3H_6O)_{d'}H$
$H_2C=CHCH_2O(C_2H_4O)_{c'}(C_3H_6O)_{d'}CH_3$
$H_2C=CHCH_2O(C_2H_4O)_{c'}C(O)CH_3$
$H_2C=C(CH_3)CH_2O(C_2H_4O)_{c'}H$
$H_2C=CC(CH_3)_2O(C_2H_4O)_{c'}H$
$H_2C=C(CH_3)CH_2O(C_2H_4O)_{c'}CH_3$
$H_2C=C(CH_3)CH_2O(C_2H_4O)_{c'}C(O)CH_3$
$H_2C=C(CH_3)CH_2O(C_2H_4O)_{c'}(C_3H_6O)_{d'}H$

$$H_2C=C(CH_3)CH_2O(C_2H_4O)_{c'}(C_3H_6O)_{d'}CH_3$$
$$H_2C=C(CH_3)CH_2O(C_2H_4O)_{c'}C(O)CH_3$$
$$HC{\equiv}CCH_2O(C_2H_4O)_{c'}H$$
$$HC{\equiv}CCH_2O(C_2H_4O)_{c'}CH_3$$
$$HC{\equiv}CCH_2O(C_2H_4O)_{c'}C(O)CH_3$$
$$HC{\equiv}CCH_2O(C_2H_4O)_{c'}(C_3H_6O)_{d'}H$$
$$HC{\equiv}CCH_2O(C_2H_4O)_{c'}(C_3H_6O)_{d'}CH_3$$
$$HC{\equiv}CCH_2O(C_2H_4O)_{c'}C(O)CH_3$$

where c' and d' are as defined above.

The polyether may also be selected from those as described in US 6,987,157, which is herein incorporated by reference for its teaching of polyethers.

Components D' or D" may be added to the silicone organic elastomer either during formation (i.e. simultaneously reacting components A), B), C) and D), in a first reaction (for example reacting a partial quantity of SiH groups of component A) with C) and D), followed by further reaction with B) or subsequently added to a formed silicone organic elastomer having SiH content (for example, from unreacted SiH units present on the silicone organic elastomer).

The amount of component D' or D" used in the hydrosilylation reaction may vary, providing the molar quantity of the total aliphatic unsaturated groups present in the reaction from components B) and D) is such that the molar ratio of the SiH units of component A) to the aliphatic unsaturated groups of components B) and D) ranges from 10/1 to 1/10.

*(ii) The Carrier Fluid*

The silicone organic elastomers (i) are contained in a carrier fluid (ii) to provide the present silicone-organic gel compositions. Typically, the carrier fluid is the solvent used for conducting the hydrosilylation reaction to form the silicone organic elastomer. Suitable carrier fluids include, organic liquids (oils and solvents), silicones and mixtures of these.

Typically, the carrier fluid is an organic liquid. Organic liquids includes those considered oils or solvents. The organic liquids are exemplified by, but not limited to, aromatic hydrocarbons, aliphatic hydrocarbons, alcohols, aldehydes, ketones, amines, esters, ethers, glycols, glycol ethers, alkyl halides and aromatic halides. Hydrocarbons include, isododecane, isohexadecane, Isopar L (C11-C13), Isopar H (C11-C12), hydrogentated polydecene. Ethers and esters include, isodecyl neopentanoate, neopentylglycol heptanoate, glycol distearate, dicaprylyl carbonate, diethylhexyl carbonate, propylene glycol n butyl ether, ethyl-3 ethoxypropionate, propylene glycol methyl ether acetate, tridecyl neopentanoate, propylene glycol methylether acetate (PGMEA), propylene glycol methylether (PGME). octyldodecyl neopentanoate, diisobutyl adipate, diisopropyl adipate, propylene glycol dicaprylate / dicaprate, and octyl palmitate. Additional organic carrier fluids suitable as a stand alone compound or as an ingredient to the carrier fluid include fats, oils, fatty acids, and fatty alcohols.

The carrier fluid may also be a low viscosity organopolysiloxane or a volatile methyl siloxane or a volatile ethyl siloxane or a volatile methyl ethyl siloxane having a viscosity at 25°C in the range of 1 to 1,000 mm$^2$/sec such as hexamethylcyclotrisiloxane, octamethylcyclotetrasiloxane, decamethylcyclopentasiloxane, dodecamethylcyclohexasiloxane, octamethyltrisiloxane, decamethyltetrasiloxane, dodecamethylpentasiloxane, tetradecamethylhexasiloxane, hexadeamethylheptasiloxane, heptamethyl-3-{(trimethylsilyl)oxy)}trisiloxane, hexamethyl-3,3,bis{(trimethylsilyl)oxy}trisiloxane pentamethyl {(trimethylsilyl)oxy} cyclotrisiloxane as well as polydimethylsiloxanes, polyethylsiloxanes, polymethylethylsiloxanes, polymethylphenylsiloxanes, polydiphenylsiloxanes.

The amount of i) silicone organic elastomer and ii) carrier fluid is such that the composition contains

2 - 95 weight percent,

alternatively 5 to 95 weight percent

alternatively 10 to 90 weight percent of i) the silicone organic elastomer, and

5 - 98 weight percent,

alternatively 95 to 5 weight percent

alternatively 90 to 10 weight percent of ii) the carrier fluid.

*Process for preparing the gel composition*

The gel compositions may be prepared by the processes of the present disclosure. The disclosed process involves;

I) reacting;

A) an organohydrogensiloxane comprising siloxy units of average formula

$$(R^1_3SiO_{0.5})_v(R^2_2SiO)_x(R^2HSiO)_y$$

wherein $R^1$ is hydrogen or $R^2$,
$R^2$ is a monovalent hydrocarbyl

$$v \geq 2, x \geq 0, y \geq 2,$$

B) a polyoxyalkylene comprising the average formula

$$R^3O-[(C_2H_4O)_c(C_3H_6O)_d(C_4H_8O)_e]-R^3$$

wherein

$R^3$ is a monovalent unsaturated aliphatic hydrocarbon group containing 2 to 12 carbon atoms,
c is from 0 to 100,
d is from 0 to 100,
e is from 0 to 100,

with a proviso the ratio of (d + e)/(c + d + e) is greater than 0.5,
C) a hydrosilylation catalyst,

and optionally

D') a hydrocarbon containing 6-30 carbons having one terminal unsaturated aliphatic hydrocarbon group,
D") a polyoxyalkylene having one terminal unsaturated aliphatic group, or mixtures of D') and D"),

in the presence of

ii) a carrier fluid,
to form a gel.

Components A), B), C), and D) and the carrier fluid ii), and the quantities used in the process are the same as described above. The order of addition of components A), B), C), and optionally D) in step I) is not critical. Typically, components A), B), and optionally D) are combined with the carrier fluid with mixing, and the mixture heated to 70-90°C. Then, the catalyst C) is added to cause the hydrosilylation reaction. Alternatively, components A) and D) are combined, mixed, and heated to 70-90°C, catalyst C) added, and subsequently component B) is added.

The process of the present disclosure may further include the step of mixing an organovinylsiloxane to the gel composition. Organovinylsiloxanes are organopolysiloxanes having at least one vinyl (Vi is $CH_2$=CH-) containing siloxy unit, that is at least one siloxy unit in the organopolysiloxane has the formula $(R_2ViSiO_{0.5})$, $(RViSiO)$, or $(ViSiO_{1.5})$. The addition of an organovinylsiloxane may enhance the long term stability of the gel composition. Although not wishing to be bound by any theory, the present inventors believe the addition of the organovinylsiloxane may react with residual SiH that may remain on the silicone organic elastomer.

*E) Personal or Healthcare Active*

Component E) is active selected from any personal or health care active. As used herein, a "personal care active" means any compound or mixtures of compounds that are known in the art as additives in the personal care formulations that are typically added for the purpose of treating hair or skin to provide a cosmetic and/or aesthetic benefit. A "healthcare active" means any compound or mixtures of compounds that are known in the art to provide a pharmaceutical or medical benefit. Thus, "healthcare active" include materials consider as an *active ingredient* or *active drug ingredient* as generally used and defined by the United States Department of Health & Human Services Food and Drug Administration, contained in Title 21, Chapter I, of the Code of Federal Regulations, Parts 200-299 and Parts 300-499.

Thus, *active ingredient* can include any component that is intended to furnish pharmacological activity or other direct effect in the diagnosis, cure, mitigation, treatment, or prevention of disease, or to affect the structure or any function of the body of a human or other animals. The phrase can include those components that may undergo chemical change in the manufacture of drug products and be present in drug products in a modified form intended to furnish the specified activity or effect.

Some representative examples of *active ingredients* include; drugs, vitamins, minerals; hormones; topical antimicrobial agents such as antibiotic *active ingredients*, antifungal *active ingredients* for the treatment of athlete's foot, jock itch, or ringworm, and acne *active ingredients*; astringent *active ingredients*; deodorant *active ingredients*; wart remover *active ingredients*; corn and callus remover *active ingredients*; pediculicide *active ingredients* for the treatment of head, pubic (crab), and body lice; *active ingredients* for the control of dandruff, seborrheic dermatitis, or psoriasis; and sunburn prevention and treatment agents.

Useful *active ingredients* for use in processes according to the invention include vitamins and its derivatives, including "pro-vitamins". Vitamins useful herein include, but are not limited to, Vitamin $A_1$, retinol, $C_2$-$C_{18}$ esters of retinol, vitamin E, tocopherol, esters of vitamin E, and mixtures thereof. Retinol includes trans-retinol, 1, 3-cis-retinol, 11-cis-retinol, 9-cis-retinol, and 3,4-didehydro-retinol, Vitamin C and its derivatives, Vitamin $B_1$, Vitamin $B_2$, Pro Vitamin B5, panthenol, Vitamin $B_6$, Vitamin $B_{12}$, niacin, folic acid, biotin, and pantothenic acid. Other suitable vitamins and the INCI names for the vitamins considered included herein are ascorbyl dipalmitate, ascorbyl methylsilanol pectinate, ascorbyl palmitate, ascorbyl stearate, ascorbyl glucocide, sodium ascorbyl phosphate, sodium ascorbate, disodium ascorbyl sulfate, potassium (ascorbyl / tocopheryl) phosphate.

RETINOL, it should be noted, is an International Nomenclature Cosmetic Ingredient Name (INCI) designated by The Cosmetic, Toiletry, and Fragrance Association (CTFA), Washington DC, for vitamin A. Other suitable vitamins and the INCI names for the vitamins considered included herein are RETINYL ACETATE, RETINYL PALMITATE, RETINYL PROPIONATE, α-TOCOPHEROL, TOCOPHERSOLAN, TOCOPHERYL ACETATE, TOCOPHERYL LINOLEATE, TOCOPHERYL NICOTINATE, and TOCOPHERYL SUCCINATE.

Some examples of commercially available products suitable for use herein are Vitamin A Acetate and Vitamin C, both products of Fluka Chemie AG, Buchs, Switzerland; COVI-OX T-50, a vitamin E product of Henkel Corporation, La Grange, Illinois; COVI-OX T-70, another vitamin E product of Henkel Corporation, La Grange, Illinois; and vitamin E Acetate, a product of Roche Vitamins & Fine Chemicals, Nutley, New Jersey.

The *active ingredient* used in processes according to the invention can be an *active drug ingredient*. Representative examples of some suitable *active drug ingredients* which can be used are hydrocortisone, ketoprofen, timolol, pilocarpine, adriamycin, mitomycin C, morphine, hydromorphone, diltiazem, theophylline, doxorubicin, daunorubicin, heparin, penicillin G, carbenicillin, cephalothin, cefoxitin, cefotaxime, 5-fluorouracil, cytarabine, 6-azauridine, 6-thioguanine, vinblastine, vincristine, bleomycin sulfate, aurothioglucose, suramin, mebendazole, clonidine, scopolamine, propranolol, phenylpropanolamine hydrochloride, ouabain, atropine, haloperidol, isosorbide, nitroglycerin, ibuprofen, ubiquinones, indomethacin, prostaglandins, naproxen, salbutamol, guanabenz, labetalol, pheniramine, metrifonate, and steroids.

Considered to be included herein as *active drug ingredients* for purposes of the present invention are antiacne agents such as benzoyl peroxide and tretinoin; antibacterial agents such as chlorohexadiene gluconate; antifungal agents such as miconazole nitrate; anti-inflammatory agents; corticosteroidal drugs; non-steroidal anti-inflammatory agents such as diclofenac; antipsoriasis agents such as clobetasol propionate; anesthetic agents such as lidocaine; antipruritic agents; antidermatitis agents; and agents generally considered barrier films.

The active component E) of the present invention can be a protein, such as an enzyme. The internal inclusion of enzymes in these compositions have advantages to prevent enzymes from deactivating and maintain bioactive effects of enzymes for longer time. Enzymes include, but are not limited to, commercially available types, improved types, recombinant types, wild types, variants not found in nature, and mixtures thereof. For example, suitable enzymes include hydrolases, cutinases, oxidases, transferases, reductases, hemicellulases, esterases, isomerases, pectinases, lactases, peroxidases, laccases, catalases, and mixtures thereof. Hydrolases include, but are not limited to, proteases (bacterial, fungal, acid, neutral or alkaline), amylases (alpha or beta), lipases, mannanases, cellulases, collagenases, lisozymes, superoxide dismutase, catalase, and mixtures thereof. Said protease include, but are not limited to, trypsin, chymotrypsin, pepsin, pancreatin and other mammalian enzymes; papain, bromelain and other botanical enzymes; subtilisin, epidermin, nisin, naringinase(L-rhammnosidase) urokinase and other bacterial enzymes. Said lipase include, but are not limited to, triacyl-glycerol lipases, monoacyl-glycerol lipases, lipoprotein lipases, e.g. steapsin, erepsin, pepsin, other mammalian, botanical, bacterial lipases and purified ones. Natural papain is preferred as said enzyme. Further, stimulating hormones, e.g. insulin, can be used together with these enzymes to boost the effectiveness of them.

Component E) may also be a sunscreen agent. The sunscreen agent can be selected from any sunscreen agent known in the art to protect skin from the harmful effects of exposure to sunlight. The sunscreen compound is typically chosen from an organic compound, an inorganic compound, or mixtures thereof that absorbs ultraviolet (UV) light. Thus, representative non limiting examples that can be used as the sunscreen agent include; Aminobenzoic Acid, Cinoxate, Diethanolamine Methoxycinnamate, Digalloyl Trioleate, Dioxybenzone, Ethyl 4-[bis(Hydroxypropyl)] Aminobenzoate, Glyceryl Aminobenzoate, Homosalate, Lawsone with Dihydroxyacetone, Menthyl Anthranilate, Octocrylene, Octyl Methoxycinnamate, Octyl Salicylate, Oxybenzone, Padimate O, Phenylbenzimidazole Sulfonic Acid, Red Petrolatum, Sulisobenzone, Titanium Dioxide, and Trolamine Salicylate, cetaminosalol, Allatoin PABA, Benzalphthalide, Benzophenone, Benzophenone 1-12, 3-Benzylidene Camphor, Benzylidenecamphor Hydrolyzed Collagen Sulfonamide, Benzylidene Camphor Sulfonic Acid, Benzyl Salicylate, Bornelone, Bumetriozole, Butyl Methoxydibenzoylmethane, Butyl PABA, Ceria/Silica, Ceria/Silica Talc, Cinoxate, DEA-Methoxycinnamate, Dibenzoxazol Naphthalene, Di-t-Butyl Hydroxybenzylidene Camphor, Digalloyl Trioleate, Diisopropyl Methyl Cinnamate, Dimethyl PABA Ethyl Cetearyldimonium Tosylate, Dioctyl Butamido Triazone, Diphenyl Carbomethoxy Acetoxy Naphthopyran, Disodium Bisethylphenyl Tiamminotriazine Stilbenedisulfonate, Disodium Distyrylbiphenyl Triaminotriazine Stilbenedisulfonate, Disodium Distyrylbiphenyl Disulfonate, Drometrizole, Drometrizole Trisiloxane, Ethyl Dihydroxypropyl PABA, Ethyl Diisopropylcinnamate, Ethyl Methoxycinnamate, Ethyl PABA, Ethyl Urocanate, Etrocrylene Ferulic Acid, Glyceryl Octanoate Dimethoxycinnamate, Glyceryl PABA, Glycol Salicylate, Homosalate, Isoamyl p-Methoxycinnamate, Isopropylbenzyl Salicylate, Isopropyl Dibenzolylmethane, Isopropyl Methoxycinnamate, Menthyl Anthranilate, Menthyl Salicylate, 4-Methylbenzylidene, Camphor, Octocrylene, Octrizole, Octyl Dimethyl PABA, Octyl Methoxycinnamate, Octyl Salicylate, Octyl Triazone, PABA, PEG-25 PABA, Pentyl Dimethyl PABA, Phenylbenzimidazole Sulfonic Acid, Polyacrylamidomethyl Benzylidene Camphor, Potassium Methoxycinnamate, Potassium Phenylbenzimidazole Sulfonate, Red Petrolatum, Sodium Phenylbenzimidazole Sulfonate, Sodium Urocanate, TEA-Phenylbenzimidazole Sulfonate, TEA-Salicylate, Terephthalylidene Dicamphor Sulfonic Acid, Titanium Dioxide, Zinc Dioxide, Serium Dioxide, TriPABA Panthenol, Urocanic Acid, and VA/Crotonates/Methacryloxybenzophenone-1 Copolymer.

Alternatively, the sunscreen agent is a cinnamate based organic compound, or alternatively, the sunscreen agent is octyl methoxycinnamate, such as Uvinul® MC 80 an ester of para-methoxycinnamic acid and 2-ethylhexanol.

Component E) may also be a fragrance or perfume. The perfume can be any perfume or fragrance active ingredient commonly used in the perfume industry. These compositions typically belong to a variety of chemical classes, as varied as alcohols, aldehydes, ketones, esters, ethers, acetates, nitrites, terpenic hydrocarbons, heterocyclic nitrogen or sulfur containing compounds, as well as essential oils of natural or synthetic origin. Many of these perfume ingredients are described in detail in standard textbook references such as Perfume and Flavour Chemicals, 1969, S. Arctander, Montclair, New Jersey.

Fragrances may be exemplified by, but not limited to, perfume ketones and perfume aldehydes. Illustrative of the perfume ketones are buccoxime; iso jasmone; methyl beta naphthyl ketone; musk indanone; tonalid/musk plus; Alpha-Damascone, Beta-Damascone, Delta-Damascone, Iso-Damascone, Damascenone, Damarose, Methyl-Dihydrojasmonate, Menthone, Carvone, Camphor, Fenchone, Alpha-Ionone, Beta-Ionone, Gamma-Methyl socalled Ionone, Fleuramone, Dihydrojasmone, Cis-Jasmone, Iso-E-Super, Methyl-Cedrenyl-ketone or Methyl- Cedrylone, Acetophenone, Methyl-Acetophenone, Para-MethoxyAcetophenone, Methyl-Beta-Naphtyl-Ketone, Benzyl-Acetone, Benzophenone, Para-Hydroxy-Phenyl-Butanone, Celery Ketone or Livescone, 6-Isopropyldecahydro-2-naphtone, Dimethyl-Octenone, Freskomenthe, 4-(1-Ethoxyvinyl)-3,3,5,5,-tetramethyl-Cyclohexanone, Methyl-Heptenone, 2-(2-(4-Methyl-3-cyclohexen-1-yl)propyl)-cyclopentanone, 1-(p-Menthen-6(2)-yl)-1-propanone, 4-(4-Hydroxy-3-methoxyphenyl)-2-butanone, 2-Acetyl-3,3-Dimethyl-Norbomane, 6,7-Dihydro-1,1,2,3,3-Pentamethyl-4(5H)-Indanone, 4-Damascol, Dulcinyl or Cassione, Gelsone, Hexalon, Isocyclemone E, Methyl Cyclocitrone, Methyl-Lavender-Ketone, Orivon, Para-tertiary-Butyl-Cyclohexanone, Verdone, Delphone, Muscone, Neobutenone, Plicatone, Veloutone, 2,4,4,7-Tetramethyl-oct-6-en-3-one, and Tetrameran.

More preferably, the perfume ketones are selected for its odor character from Alpha Damascone, Delta Damascone, Iso Damascone, Carvone, Gamma-Methyl-Ionone, Iso-E-Super, 2,4,4,7-Tetramethyl-oct-6-en-3-one, Benzyl Acetone, Beta Damascone, Damascenone, methyl dihydrojasmonate, methyl cedrylone, and mixtures thereof.

Preferably, the perfume aldehyde is selected for its odor character from adoxal; anisic aldehyde; cymal; ethyl vanillin; florhydral; helional; heliotropin; hydroxycitronellal; koavone; lauric aldehyde; lyral; methyl nonyl acetaldehyde; P. T. bucinal; phenyl acetaldehyde; undecylenic aldehyde; vanillin; 2,6,10-trimethyl-9-undecenal, 3-dodecen-1-al, alphan-amyl cinnamic aldehyde, 4-methoxybenzaldehyde, benzaldehyde, 3-(4-tert butylphenyl)-propanal, 2-methyl-3-(para-methoxyphenyl propanal, 2-methyl-4-(2,6,6-trimethyl-2(1)-cyclohexen-1-yl) butanal, 3-phenyl-2-propenal, cis-/trans-3,7-dimethyl-2,6-octadien-1-al, 3,7-dimethyl-6-octen-1-al, [(3,7-dimethyl-6-octenyl)oxy] acetaldehyde, 4-isopropylbenzyaldehyde, 1,2,3,4,5,6,7,8-octahydro-8,8-dimethyl-2-naphthaldehyde, 2,4-dimethyl-3-cyclohexen-1-carboxaldehyde, 2-methyl-3-(isopropylphenyl)propanal, 1-decanal; decyl aldehyde, 2,6-dimethyl-5-heptenal, 4-(tricyclo[5.2.1.0(2,6)]-decylidene-8)-butanal, octahydro-4,7-methano-1H- indenecarboxaldehyde, 3-ethoxy-4-hydroxy benzaldehyde, para-ethyl-alpha, alpha-dimethyl hydrocinnamaldehyde, alpha-methyl-3,4-(methylenedioxy)-hydrocinnamaldehyde, 3,4-methylenedioxybenzaldehyde, alpha-n-hexyl cinnamic aldehyde, m-cymene-7-carboxaldehyde, alpha-methyl phenyl acetaldehyde, 7-hydroxy-3,7-dimethyl octanal, Undecenal, 2,4,6-trimethyl-3-cyclohexene-1-carboxaldehyde, 4-(3)(4-methyl-3-pentenyl)-3-cyclohexen-carboxaldehyde, 1-dodecanal, 2,4-dimethyl cyclohexene-3-carboxaldehyde, 4-(4-hydroxy-4-methyl pentyl)-3-cylohexene-1-carboxaldehyde, 7-methoxy-3,7-dimethyloctan-1-al, 2-methyl undecanal, 2-methyl decanal, 1-nonanal, 1-octanal, 2,6,10-trimethyl-5,9-undecadienal, 2-methyl-3-(4-tertbutyl)propanal, dihydrocinnamic aldehyde, 1-methyl-4-(4-methyl-3-pentenyl)-3-cyclohexene-1-carbox aldehyde, 5 or 6 methoxyl 0 hexahydro-4,7-methanoindan-1 or 2- carboxaldehyde, 3,7-dimethyloctan-1-al, 1 -undecanal, 10-undecen-1-al, 4-hydroxy-3-methoxy benzaldehyde, 1-methyl-3-(4-methylpentyl)-3-cyclhexenecarboxaldehyde, 7-hydroxy-3,7-dimethyl-octanal, trans-4-decenal, 2,6-nonadienal, paratolylacetaldehyde; 4-methylphenylacetaldehyde, 2-methyl-4-(2,6,6-trimethyl-1-cyclohexen-1-yl)-2-butena 1, ortho-methoxycinnamic aldehyde, 3,5,6-trimethyl-3-cyclohexene carboxaldehyde, 3,7-dimethyl-2-methylene-6-octenal, phenoxyacetaldehyde, 5,9-dimethyl-4,8-decadienal, peony aldehyde (6,10-dimethyl-3-oxa-5,9-undecadien-1-al), hexahydro-4,7-methanoindan-1-carboxaldehyde, 2-methyl

octanal, alpha-methyl-4-(1-methyl ethyl) benzene acetaldehyde, 6,6-dimethyl-2-norpinene-2-propionaldehyde, para methyl phenoxy acetaldehyde, 2-methyl-3-phenyl-2-propen-1-al, 3,5,5-trimethyl hexanal, Hexahydro-8,8-dimethyl-2-naphthaldehyde, 3-propyl-bicyclo[2.2.1]-hept-5-ene-2-carbaldehyde, 9-decenal, 3-methyl-5-phenyl-1-pentanal, methylnonyl acetaldehyde, hexanal, trans-2-hexenal, 1-p-menthene-q-carboxaldehyde and mixtures thereof.

More preferred aldehydes are selected for their odor character from 1-decanal, benzaldehyde, florhydral, 2,4-dimethyl-3-cyclohexen-1-carboxaldehyde; cis/trans-3,7-dimethyl-2,6-octadien-1-al; heliotropin; 2,4,6-trimethyl-3-cyclohexene-1-carboxaldehyde; 2,6-nonadienal; alpha-n-amyl cinnamic aldehyde, alpha-n-hexyl cinnamic aldehyde, P.T. Bucinal, lyral, cymal, methyl nonyl acetaldehyde, hexanal, trans-2-hexenal, and mixture thereof.

In the above list of perfume ingredients, some are commercial names conventionally known to one skilled in the art, and also includes isomers. Such isomers are also suitable for use in the present invention.

Component E) may also be one or more plant extract. Examples of these components are as follows: Ashitaba extract, avocado extract, hydrangea extract, Althea extract, Arnica extract, aloe extract, apricot extract, apricot kernel extract, Ginkgo Biloba extract, fennel extract, turmeric[Curcuma] extract, oolong tea extract, rose fruit extract, Echinacea extract, Scutellaria root extract, Phellodendro bark extract, Japanese Coptis extract, Barley extract, Hyperium extract, White Nettle extract, Watercress extract, Orange extract, Dehydrated saltwater, seaweed extract, hydrolyzed elastin, hydrolyzed wheat powder, hydrolyzed silk, Chamomile extract, Carrot extract, Artemisia extract, Glycyrrhiza extract, hibiscustea extract, Pyracantha Fortuneana Fruit extract, Kiwi extract, Cinchona extract, cucumber extract, guanocine, Gardenia extract, Sasa Albo-marginata extract, Sophora root extract, Walnut extract, Grapefruit extract, Clematis extract, Chlorella extract, mulberry extract, Gentiana extract, black tea extract, yeast extract, burdock extract, rice bran ferment extract, rice germ oil, comfrey extract, collagen, cowberry extract, Gardenia extract, Asiasarum Root extract, Family of Bupleurum extract, umbilical cord extract, Salvia extract, Saponaria extract, Bamboo extract, Crataegus fruit extract, Zanthoxylum fruit extract, shiitake extract, Rehmannia root extract, gromwell extract, Perilla extract, linden extract, Filipendula extract, peony extract, Calamus Root extract, white birch extract, Horsetail extract,Hedera Helix(Ivy) extract, hawthorn extract, Sambucus nigra extract, Achillea millefolium extract, Mentha piperita extract, sage extract, mallow extract, Cnidium officinale Root extract, Japanese green gentian extract, soybean extract, jujube extract, thyme extract, tea extract, clove extract, Gramineae imperata cyrillo extract, Citrus unshiu peel extract Japanese Angellica Root extract, Calendula extract, Peach Kernel extract, Bitter orange peel extract, Houttuyna cordata extract, tomato extract, natto extract, Ginseng extract, Green tea extract (camelliea sinesis), garlic extract, wild rose extract, hibiscus extract, Ophiopogon tuber extarct, Nelumbo nucifera extract, parsley extract, honey, hamamelis extract, Parietaria extract, Isodonis herba extract, bisabolol extract, Loquat extract, coltsfoot extract, butterbur extract, Porid cocos wolf extract, extract of butcher's broom, grape extract, propolis extract, luffa extract, safflower extract, peppermintextract, linden tree extract, Paeonia extract, hop extract, pine tree extract, horse chestnut extract, Mizu-bashou [Lysichiton camtschatcese]extract, Mukurossi peel extract, Melissa extract, peach extract, cornflower extract, eucalyptus extract, saxifrage extract, citron extract, coix extract, mugwort extract, lavender extract, apple extract, lettuce extract, lemon extract, Chinese milk vetch extract, rose extract, rosemary extract, Roman Chamomile extract, and royal jelly extract.

The amount of component E) present in the silicone gel composition may vary, but typically range as follows; .05 to 50 wt%, alternatively 1 to 25 wt %, or alternatively 1 to 10 wt%,
based on the amount by weight of silicone elastomer gel present in the composition, that is total weight of components A), B), C) and D) in the silicone gel composition.

The active, component E), may be added to the silicone gel composition either during the making of the silicone elastomer (pre-load method), or added after the formation of the silicone elastomer gel (post load method).

*Gel Paste compositions containing the Silicone organic elastomer*

The gel compositions of the present invention can be used to prepare gel paste compositions by;

I) shearing the silicone organic elastomer gel, as described above,
II) combining the sheared silicone organic elastomer gel with additional quantities of
ii) the carrier fluid, as described above, and optionally
E) a personal or health care active active to form a gel paste composition.

The silicone organic elastomer gel compositions of the present invention may be considered as discrete crosslinked silicone organic elastomers dispersed in carrier fluids. The silicone organic elastomer gel compositions are also effective rheological thickeners for many organic and silicone fluids. As such they can be used to prepare useful gel blend compositions, such as "paste" compositions.

To make such silicone organic elastomer pastes, the aforementioned silicone organic elastomer gels of known initial elastomer content are sheared to obtain small particle size may optionally be further diluted to a final elastomer content. "Shearing", as used herein refers to any shear mixing process, such as obtained from homogenizing, sonalating, or any other mixing processes known in the art as shear mixing. The shear mixing of the silicone organic elastomer gel composition results in a composition having reduced particle size. The subsequent composition having reduced particle size is then further combined with additional quantities of ii) the carrier fluid. Typically, the amount of carrier fluid added to the gel to form the gel paste is sufficient to provide a gel paste composition containing 30 wt % of the silicone organic elastomer, alternatively 20 wt %, or alternatively 10 wt%. The carrier fluid may be any carrier fluid as described above, but typically is a aliphatic hydrocarbon. The technique for combining the ii) the carrier fluid with the silicone organic elastomer composition, and typically involves simple stirring or mixing. The resulting compositions may be considered as a paste, having a viscosity at least 50 Pa·s, alternatively at least 100 Pa·s, or alternatively at least 200 Pa·s, as measured on a Brookfield DVII+ viscometer with Helipath attachment using spindle T-D (20.4 mm crossbar) at 2.5 rpm.

Alternatively, gel paste compositions may be formed by;

I) preparing the silicone organic elastomer gel (as described above) under shear,
II) combining the sheared silicone organic elastomer gel with additional quantities of
ii) the carrier fluid, as described above, and optionally
E) a personal or health care active active

to form a gel paste composition.

*F) Additional Optional components*

Composition according to the invention may also contain a number of optional ingredients. In particular, these optional components are selected from those known in the state of the art to be ingredient in personal care formulations. Illustrative, non-limiting examples include; surfactants, solvents, powders, coloring agents, thickeners, waxes, stabilizing agents, pH regulators, and silicones.

Thickening agent may be added to provide a convenient viscosity. For example, viscosities within the range of 500 to 25,000 $mm^2$/s at 25°C or more alternatively in the range of 3,000 to 7,000 $mm^2$/s are usually suitable. Suitable thickening agents are exemplified by sodium alginate, gum arabic, polyoxyethylene, guar gum, hydroxypropyl guar gum, ethoxylated alcohols, such as laureth-4 or polyethylene glycol 400, cellulose derivatives exemplified by methylcellulose, methylhydroxypropylcellulose, hydroxypropylcellulose, polypropylhydroxyethylcellulose, starch, and starch derivatives exemplified by hydroxyethylamylose and starch amylose, locust bean gum, electrolytes exemplified by sodium chloride and ammonium chloride, and saccharides such as fructose and glucose, and derivatives of saccharides such as PEG-120 methyl glucose diolate or mixtures of 2 or more of these. Alternatively the thickening agent is selected from cellulose derivatives, saccharide derivatives, and electrolytes, or from a combination of two or more of the above thickening agents exemplified by a combination of a cellulose derivative and any electrolyte, and a starch derivative and any electrolyte. The thickening agent, where used is present in the shampoo compositions of this invention in an amount sufficient to provide a viscosity in the final shampoo composition of from 500 to 25,000 $mm^2$/s. Alternatively the thickening agent is present in an amount from about 0.05 to 10 wt% and alternatively 0.05 to 5 wt% based on the total weight of the composition.

Stabilizing agents can be used in the water phase of the compositions. Suitable water phase stabilizing agents can include alone or in combination one or more electrolytes, polyols, alcohols such as ethyl alcohol, and hydrocolloids. Typical electrolytes are alkali metal salts and alkaline earth salts, especially the chloride, borate, citrate, and sulfate salts of sodium, potassium, calcium and magnesium, as well as aluminum chlorohydrate, and polyelectrolytes, especially hyaluronic acid and sodium hyaluronate. When the stabilizing agent is, or includes, an electrolyte, it amounts to about 0.1 to 5 wt % and more alternatively 0.5 to 3 wt % of the total composition. The hydrocolloids include gums, such as Xantham gum or Veegum and thickening agents, such as carboxymethyl cellulose. Polyols, such as glycerine, glycols, and sorbitols can also be used. Alternative polyols are glycerine, propylene glycol, sorbitol and butylene glycol. If a large amount of a polyol is used, one need not add the electrolyte. However, it is typical to use a combination of an electrolyte, a polyol and an hydrocolloid to stabilize the water phase, e.g. magnesium sulfate, butylene glycol and Xantham gum.

Other additives can include powders and pigments especially when the composition according to the invention is intended to be used for make-up. The powder component of the invention can be generally defined as dry, particulate matter having a particle size of 0.02-50 microns. The particulate matter may be colored or non-colored (for example white). Suitable powders include but not limited to bismuth oxychloride, titanated mica, fumed silica, spherical silica beads, polymethylmethacrylate beads, , boron nitride, aluminum silicate, aluminum starch octenylsuccinate, bentonite, kaolin, magnesium aluminum silicate, silica, talc, mica, titanium dioxide, kaolin, nylon, silk powder. The above mentioned powders may be surface treated to render the particles hydrophobic in nature.

The powder component also comprises various organic and inorganic pigments. The organic pigments are generally various aromatic types including azo, indigoid, triphenylmethane, anthraquinone, and xanthine dyes which are designated as D&C and FD&C blues, browns, greens, oranges, reds, yellows, etc. Inorganic pigments generally consist of insoluble metallic salts of certified color additives, referred to as the Lakes or iron oxides. A pulverulent coloring agent, such as carbon black, chromium or iron oxides, ultramarines, manganese pyrophosphate, iron blue, and titanium dioxide, pearlescent agents, generally used as a mixture with colored pigments, or some organic dyes, generally used as a mixture with colored pigments and commonly used in the cosmetics industry, can be added to the composition. In general, these coloring agents can be present in an amount by weight from 0 to 20% with respect to the weight of the final composition. Pulverulent inorganic or organic fillers can also be added, generally in an amount by weight from 0 to 40% with respect to the weight of the final composition. These pulverulent fillers can be chosen from talc, micas, kaolin, zinc or titanium oxides, calcium or magnesium carbonates, silica, spherical titanium dioxide, glass or ceramic beads, metal soaps derived from carboxylic acids having 8-22 carbon atoms, non-expanded synthetic polymer powders, expanded powders and powders from natural organic compounds, such as cereal starches, which may or may not be crosslinked, copolymer microspheres such as EXPANCEL (Nobel Industrie), polytrap and silicone resin microbeads (TOSPEARL from Toshiba, for example).

The waxes or wax-like materials useful in the composition according of the invention have generally have a melting point range of 35 to 120°C at atmospheric pressure. Waxes in this category include synthetic wax, ceresin, paraffin, ozokerite, beeswax, carnauba, microcrystalline, lanolin, lanolin derivatives, candelilla, cocoa butter, shellac wax, spermaceti, bran wax, capok wax, sugar cane wax, montan wax, whale wax, bayberry wax, or mixtures thereof. Mention may be made, among the waxes capable of being used as non-silicone fatty substances, of animal waxes, such as beeswax; vegetable waxes, such as carnauba, candelilla wax ; mineral waxes, for example paraffin or lignite wax or microcrystalline waxes or ozokerites; synthetic waxes, including polyethylene waxes, and waxes obtained by the Fischer-Tropsch synthesis. Mention may be made, among the silicone waxes, of polymethylsiloxane alkyls, alkoxys and/or esters.

Such optional components include other silicones (including any already described above), organofunctional siloxanes, alkylmethylsiloxanes, siloxane resins and silicone gums.

Alkylmethylsiloxanes may be included in the present compositions. These siloxane polymers generally will have the formula $Me_3SiO[Me_2SiO]_y[MeRSiO]_zSiMe_3$, in which R is a hydrocarbon group containing 6-30 carbon atoms, Me represents methyl, and the degree of polymerization (DP), i.e., the sum of y and z is 3-50. Both the volatile and liquid species of alkymethysiloxanes can be used in the composition.

Silicone gums may be included in the present compositions. Polydiorganosiloxane gums are known in the art and are available commercially. They consist of generally insoluble polydiorganosiloxanes having a viscosity in excess of 1,000,000 centistoke ($mm^2$/s) at 25 °C., alternatively greater than 5,000,000 centistoke ($mm^2$/s) at 25 °C. These silicone gums are typically sold as compositions already dispersed in a suitable solvent to facilitate their handling. Ultra-high viscosity silicones can also be included as optional ingredients. These ultra-high viscosity silicones typically have a kinematic viscosity greater than 5 million centistoke ($mm^2$/s) at 25 °C, to about 20 million centistoke ($mm^2$/s) at 25 °C. Compositions of this type in the form of suspensions are most preferred, and are described for example in US Patent 6.013,682 (January 11, 2000).

Silicone resins may be included in the present compositions. These resin compositions are generally highly crosslinked polymeric siloxanes. Crosslinking is obtained by incorporating trifunctional and/or tetrafunctional silanes with the monofunctional silane and/or difunctional silane monomers used during manufacture. The degree of crosslinking required to obtain a suitable silicone resin will vary according to the specifics of the silane monomer units incorporated during manufacture of the silicone resin. In general, any silicone having a sufficient level of trifunctional and tetrafunctional siloxane monomer units, and hence possessing sufficient levels of crosslinking to dry down to a rigid or a hard film can be considered to be suitable for use as the silicone resin. Commercially available silicone resins suitable for applications herein are generally supplied in an unhardened form in low viscosity volatile or nonvolatile silicone fluids. The silicone resins should be incorporated into compositions of the invention in their non-hardened forms rather than as hardened resinous structures.

Silicone carbinol Fluids may be included in the present compositions. These materials are described in WO 03/101412 A2, and can be commonly described as substituted hydrocarbyl functional siloxane fluids or resins.

Water soluble or water dispersible silicone polyether compositions may be included in the present compositions: These are also known as polyalkylene oxide silicone copolymers, silicone poly(oxyalkylene) copolymers, silicone glycol copolymers, or silicone surfactants. These can be linear rake or graft type materials, or ABA type where the B is the siloxane polymer block, and the A is the poly(oxyalkylene) group. The poly(oxyalkylene) group can consist of polyethylene oxide, polypropylene oxide, or mixed polyethylene oxide/polypropylene oxide groups. Other oxides, such as butylene oxide or phenylene oxide are also possible.

Compositions according to the invention can be used in w/o, w/s, or multiple phase emulsions using silicone emulsifiers. Typically the water-in-silicone emulsifier in such formulation is non-ionic and is selected from polyoxyalkylene-substituted silicones, silicone alkanolamides, silicone esters and silicone glycosides. Suitable silicone-based surfactants are well known in the art, and have been described for example in US 4,122,029 (Gee et al.), US 5,387,417 (Rentsch), and US 5,811,487 (Schulz et al).

When the composition according to the invention is an oil-in-water emulsion, it will include common ingredients generally used for preparing emulsions such as but not limited to non ionic surfactants well known in the art to prepare o/w emulsions . Examples of nonionic surfactants include polyoxyethylene alkyl ethers, polyoxyethylene alkylphenol ethers, polyoxyethylene lauryl ethers, polyoxyethylene sorbitan monoleates, polyoxyethylene alkyl esters, polyoxyethylene sorbitan alkyl esters, polyethylene glycol, polypropylene glycol, diethylene glycol, ethoxylated trimethylnonanols, and polyoxyalkylene glycol modified polysiloxane surfactants.

The composition according to the invention can also be under the form of aerosols in combination with propellant gases, such as carbon dioxide, nitrogen, nitrous oxide, volatile hydrocarbons such as butane, isobutane, or propane and chlorinated or fluorinated hydrocarbons such as dichlorodifluoromethane and dichlorotetrafluoroethane or dimethylether.

The silicone elastomer gel compositions can be used in a variety of personal, household, and healthcare applications. In particular, the compositions of the present invention may be used: as thickening agents, as taught in US Patent Nos. 6,051,216, 5,919,441, 5,981,680; to structure oils, as disclosed in WO 2004/060271 and WO 2004/060101; in sunscreen compositions as taught in WO 2004/060276; as structuring agents in cosmetic compositions also containing film-forming resins, as disclosed in WO 03/105801; in the cosmetic compositions as taught in US Patent Application Publications 2003/0235553, 2003/0072730, 2003/0170188, EP 1,266,647, EP 1,266,648, EP1,266,653, WO 03/105789, WO 2004/000247 and WO 03/106614; as structuring agents as taught in WO 2004/054523; in long wearing cosmetic compositions as taught in US Patent Application Publication 2004/0180032; in transparent or translucent care and/or make up compositions as discussed in WO 2004/054524; all of which are incorporated herein by reference.

Silicone elastomer gels can also be used in anti-perspirant and deodorant compositions under but not limited to the form of sticks, soft solid, roll on, aerosol, and pumpsprays. Some examples of antiperspirant agents and deodorant agents are Aluminum Chloride, Aluminum Zirconium Tetrachlorohydrex GLY, Aluminum Zirconium Tetrachlorohydrex PEG, Aluminum Chlorohydrex, Aluminum Zirconium Tetrachlorohydrex PG, Aluminum Chlorohydrex PEG, Aluminum Zirconium Trichlorohydrate, Aluminum Chlorohydrex PG, Aluminum Zirconium Trichlorohydrex GLY, Hexachlorophene, , Benzalkonium Chloride,, Aluminum Sesquichlorohydrate, Sodium Bicarbonate, Aluminum Sesquichlorohydrex PEG, ,Chlorophyllin-Copper Complex, Triclosan, Aluminum Zirconium Octachlorohydrate, and Zinc Ricinoleate.

The personal care compositions of this invention may be in the form of a cream, a gel, a powder, a paste, or a freely pourable liquid. Generally, such compositions can generally be prepared at room temperature if no solid materials at room temperature are presents in the compositions, using simple propeller mixers, Brookfield counter-rotating mixers, or homogenizing mixers. No special equipment or processing conditions are typically required. Depending on the type of form made, the method of preparation will be different, but such methods are well known in the art.

The compositions according to this invention can be used by the standard methods, such as applying them to the human body, e.g. skin or hair, using applicators, brushes, applying by hand, pouring them and/or possibly rubbing or massaging the composition onto or into the body. Removal methods, for example for colour cosmetics are also well known standard methods, including washing, wiping, peeling and the like. For use on the skin, the compositions according to the present invention may be used in a conventional manner for example for conditioning the skin. An effective amount of the composition for the purpose is applied to the skin. Such effective amounts generally range from about 1mg/cm$^2$ to about 3 mg/cm$^2$. Application to the skin typically includes working the composition into the skin. This method for applying to the skin comprises the steps of contacting the skin with the composition in an effective amount and then rubbing the composition into the skin. These steps can be repeated as many times as desired to achieve the desired benefit.

The use of the compositions according to the invention on hair may use a conventional manner for conditioning hair. An effective amount of the composition for conditioning hair is applied to the hair. Such effective amounts generally range from about 1g to about 50g, preferably from about 1g to about 20g. Application to the hair typically includes working the composition through the hair such that most or all of the hair is contacted with the composition. This method for conditioning the hair comprises the steps of applying an effective amount of the hair care composition to the hair, and then working the composition through the hair. These steps can be repeated as many times as desired to achieve the desired conditioning benefit. When a high silicone content is incorporated in a hair care composition according to the invention, this may be a useful material for split end hair products.

The compositions according to this invention can be used on the skin of humans or animals for example to moisturize, color or generally improve the appearance or to apply actives, such as sunscreens, deodorants, insect repellents etc.

Examples

These examples are intended to illustrate the invention to one of ordinary skill in the art and should not be interpreted as limiting the scope of the invention set forth in the claims. All measurements and experiments were conducted at 23°C, unless indicated otherwise.

Materials

*Organohydrogensiloxane 1* = a dimethyl, methylhydrogen polysiloxane having an average formula of $(CH_3)_3SiO[(CH_3)_2SiO]_x[(CH_3)HSiO]_ySi(CH_3)_3$, where x and y are of a value such that the organohydrogensiloxane has a viscosity of 116 $mm^2$/s (cSt) at 23°C and contains 0.084 wt. % H as Si-H.

*Organohydrogensiloxane 2* = a dimethyl, methylhydrogen polysiloxane having an average formula of $(CH_3)_3SiO[(CH_3)_2SiO]_x[(CH_3)HSiO]_ySi(CH_3)_3$, where x and y are of a value such that the organohydrogensiloxane has a viscosity of 67 (cSt) at 23°C and contains 0.15 wt. % H as Si-H.

*Oiganovinylsiloxane* = a vinyl terminal dimethyl polysiloxane having the formula $(ViMe_2SiO_{0.5})_2(Me_2SiO)_x$ where x is of a value such that the organovinylsiloxane has a viscosity of 5 $mm^2$/s (cSt) at 23°C.

*Polvalkyloxylenes* having either one or two terminal allyl groups and varying amounts of propylene or ethylene oxide units were used in these example as summarized in the table below.

| Polyalkyloxylene # | Average structure | MW (g/mol) | Vinyl concentration (mmol/g) |
|---|---|---|---|
| *Polyalkyloxylene 1* | $CH_2=CHCH_2O(CH_3CH(CH_3)O)_{7.05}CH_2CH=CH_2$ | 505.9 | 3.95 |
| *Polyalkyloxylene 2* | $CH_2=CHCH_2O(CH_3CH(CH_3)O)_{17.3}CH_2CH=CH_2$ | 1086.5 | 1.84 |
| *Polyalkyloxylene 3* | $CH_2=CHCH_2O(CH_3CH(CH_3)O)_{34.6}CH_2CH=CH_2$ | 2073.9 | 0.96 |
| *Polyalkyloxylene 4* | $CH_2=CHCH_2O(CH_3CH(CH_3)O)_{17}OBu$ | 1134.7 | 1.97 |
| *Polyalkyloxylene 5* | $CH_2=CHCH_2O(CH_2CH_2O)_{17}OCH_3$ | 837.0 | 5.06 |

The di-allyl functional polyalkylene oxides were synthesized from the corresponding OH terminal polyalkylene oxides by reaction with NaH to form the alkoxide, which was then reacted with allyl chloride to form the allyl terminal polyether. Alternately, the di-allyl functional polyalkylene oxides may be purchased commercially.

Example 1

*Reparation of a Gel containing Polyalhyloxylene Crosslinked Silicone organic elastomer*

First, 42.40 g (35.5 mmol Si-H) of *Organohydrogensiloxane 1,* 9.83 g of *Polvalhyloxylene 1*, and 194 g of isododecane were weighed into a 16 oz wide mouth jar containing a teflon coated stir bar. Then, the jar was sealed and heated to 70°C using either a water bath or oven The jar was removed from heat, and then while stirring, 0.6 g of SYLOFF 4000 catalyst (0.52 wt. % platinum) was added to provide 12 ppm platinum. The jar was capped, place in a 70°C water bath, and stirring continued until the reaction mixture gelled. The mixture was held at 70°C in either a water bath or oven for an additional 3 hours to provide a gel containing 21.1% silicone-organic elastomer.

Gels containing polyalkyloxylene crosslinked silicone organic elastomers having varying amounts of propylene oxide units (17.3 or 34.6) were synthesized using the procedure as described above. The formulations used to prepare these gel compositions are summarized in Table 1.

**Table 1**

| Example # | 1A | 1B | 1C | 2A | 2B |
|---|---|---|---|---|---|
| <u>Gel Formulations</u> | | | | | |
| *Organohydrogensiloxane1* | 42.40 g | 34.94 g | 26.86 g | 34.15 g | 27.67 g |
| *Polyalkyloxylene 1* | 9.83 g | | | 8.82 g | |
| *Polvalhyloxylene 2* | | 17.26 g | | | 15.30 g |
| *Polyalkyloxylene 3* | | | 25.34 g | | |
| Isododecane | 194.4 g | 194.4 g | 194.4 g | 225 g | 225 g |
| Syloff 4000 - Pt catalyst | 0.6 g | 0.6 g | 0.6 g | 0.6 g | 0.6 g |

<u>Example 2 (comparative)</u>

*Reparation of a Gel containing Hexadiene Crosslinked Silicone Organic elastomer*

First, 50.32 g (42.2 mmol Si-H) of *Organohydrogensiloxane 1,* 1.88 g (46.3 meq unsaturation) of 1,5-hexadiene, and 194 g of isododecane were weighed into a 16 oz wide mouth jar containing a stir bar. The 8.5 % excess of olefin (Si-H:Vi = 0.92) was intended to minimize the amount of residual Si-H. The jar was sealed and then heated to 70°C in an oven. The jar was removed from heat, and then while stirring, 0.6 g of SYLOFF 4000 catalyst (0.52 wt. % platinum) was added. This brought the mixture up to 12 ppm platinum. The jar was capped and then placed in a 70°C water bath where stirring continued until gelation. The mixture was held at 70°C in an oven for 3 hours to form the gel containing 21.1 % elastomer.

<u>Example 3</u>

*Reparation of Gel Pastes from Elastomer Gels*

The elastomer gels, as made in Example 1 and Example 2, were made into gel pastes using high shear mixing. The shear steps included the addition of isododecane, *organovinylsiloxane,* and octyl methoxycinnamate (OMC) to produce the pastes shown in Tables 2-5. The materials in Table 2 were sheared in a Waring Commercial Laboratory Blender. In shear step 1, the gel was sheared for 20 seconds at setting 1, then 20 seconds at setting 3, then 20 seconds at setting 5. Isododecane and an organovinylsiloxane were added followed by shearing for 30 seconds at each of the following settings: 1, 2, 3, 3. Between each setting, the material was scraped from the sides of the mixer cup using a spatula. Before the third shear step, additional isododecane and octyl methoxycinnamate were added according to Table 2 followed by 20 second shearing at each of the following settings: 1, 1, 1. Between each setting, the material was scraped from the sides of the mixer cup. The materials in Tables 3-5 were sheared using a Hauschild Speed Mixer model DAC 150 FVZ purchased from FlackTek Inc. In the first shear step, the gel was sheared for 25 seconds on the maximum setting (approximately 3500rpm). Isododecane and an organovinylsiloxane were added followed by shearing for 25 seconds on the maximum setting. Additional isododecane and octyl methoxycinnamate were added and the material was sheared for 25 seconds on the maximum setting. The materials is Table 6, were processed in the same manner as in Tables 3-5, except the two shear steps were 30 seconds in duration rather than 25 seconds.

**Table 2: Hexadiene-Crosslinked Silicone organic elastomer Paste Processing with Addition of OMC**

| Elastomer Blend Paste Processing | | 0%v OMC | 6%v OMC | 12%v OMC | 18%v OMC |
|---|---|---|---|---|---|
| shear step 1 | Example 2 gel (g) | 59.3 | 59.3 | 59.3 | 59.3 |
| shear step 2 | isododecane (g) | 4.1 | 4.1 | 4.1 | 4.1 |
| | organovinylsiloxane (g) | 0.5 | 0.5 | 0.5 | 0.5 |
| shear step 3 | octyl methoxycinnamate (g) | 0.0 | 6.2 | 12.3 | 18.5 |
| | isododecane (g) | 15.0 | 10.4 | 5.8 | 1.3 |

**Table 3: Diallyl Polyether-Crosslinked (7DP) Silicone organic elastomer Paste Processing with Addition of OMC**

| Elastomer Blend Paste Processing | | 0%v OMC | 6%v OMC | 12%v OMC | 18%v OMC |
|---|---|---|---|---|---|
| shear step 1 | Example 1A gel (g) | 59.7 | 59.7 | 59.7 | 59.7 |
| shear step 2 | isododecane (g) | 4.1 | 4.1 | 4.1 | 4.2 |
| | organovinylsiloxane (g) | 0.5 | 0.5 | 0.5 | 0.5 |
| shear step 3 | octyl methoxycinnamate (g) | 0.0 | 6.3 | 12.4 | 18.5 |
| | isododecane (g) | 15.0 | 10.4 | 5.8 | 1.3 |

**Table 4: Diallyl Polyether-Crosslinked (17DP) Silicone organic elastomer Paste Processing with Addition of OMC**

| Elastomer Blend Paste Processing | | 0%v OMC | 6%v OMC | 12%v OMC | 18%v OMC |
|---|---|---|---|---|---|
| shear step 1 | Example 1 B gel (g) | 59.8 | 59.8 | 59.8 | 59.8 |
| shear step 2 | isododecane (g) | 4.1 | 4.1 | 4.1 | 4.1 |
| | organovinylsiloxane (g) | 0.5 | 0.5 | 0.5 | 0.5 |
| shear step 3 | octyl methoxycinnamate (g) | 0.0 | 6.2 | 12.4 | 18.5 |
| | isododecane (g) | 15.0 | 10.5 | 5.8 | 1.3 |

**Table 5: Diallyl Polyether-Crosslinked (35DP) Silicone organic elastomer Paste Processing with Addition of OMC**

| Elastomer Blend Paste Processing | | 0%v OMC | 6%v OMC | 12%v OMC | 18%v OMC |
|---|---|---|---|---|---|
| shear step 1 | Example 1C gel (g) | 59.7 | 59.7 | 59.8 | 59.7 |
| shear step 2 | isododecane (g) | 4.1 | 4.1 | 4.1 | 4.1 |
| | organovinylsiloxane (g) | 0.5 | 0.5 | 0.5 | 0.5 |
| shear step 3 | octyl methoxycinnamate (g) | 0.0 | 6.2 | 12.3 | 18.5 |
| | isododecane (g) | 15.0 | 10.4 | 5.9 | 1.3 |

**Table 6: Diallyl Polyether-Crosslinked Silicone organic elastomer Paste Processing**

| | Paste 2B | Paste 2A |
|---|---|---|
| Gel 2A | | 63.75g |
| Gel 2B | 63.75 g | |
| isododecane | 21.26g | 21.26g |
| organovinylsiloxane | 0.43g | 0.43g |

Example 4

*Compatibility of Gels containing a Polvalhyloxylene Crosslinked Silicone organic elastomer with octyl methoxycinnamate*

Organic compatibility of the silicone organic elastomer gels may be improved by increasing the degree of polymerization (DP) of the polypropylene glycol (PPG) crosslinker, as demonstrated in this example. When using the same organohydrogensiloxane, increasing the number of propylene oxide units in the polyalkoxylene increases the organic content of the elastomeric component and organic compatibility, as demonstrated in this example using octyl methoxycinnamate, an organic sunscreen known to have limited solubility with silicone organic elastomer gels. As summarized in Table 7, the compatibility of the silicone organic elastomer with octyl methoxycinnamate increases as the propylene oxide content increases (as denoted by DP or degree of polymerization, i.e. number of propylene oxide units), as based on clarity.

Viscosity changes also provided an indication of compatibility. As shown in Table 8, viscosity decreases with higher octyl methoxycinnamate levels in the hexadiene-crosslinked elastomer pastes while viscosity was maintained or increased in the polypropylene glycol crosslinked elastomer pastes. The viscosities were determined on a Brookfield DV-II+ Rheometer with the Helipath attachment and T-D spindle (t-bar geometry) at 2.5 RPM. Viscosities were determined one day after the samples were synthesized. The samples were vacuum de-aired and allowed to sit undisturbed for a minimum of four hours prior to testing. The data were acquired during two cycles of a down and up path through the sample. The reported viscosity was an average of the first upward and second downward pass of the t-bar through the sample.

**Table 7**

**Compatibility (Clarity) of Elastomer Blends as a Function of the Crosslinker and Volume Percent OMC**

| Crosslinker | Volume % OMC | | | |
| --- | --- | --- | --- | --- |
| | 0 | 6 | 12 | 18 |
| Hexadiene | A | B | D | E |
| 7 DP PPG | A | A | A | C |
| 17 DP PPG | A | A | A | B |
| 34 DP PPG | A | A | A | A |

*A = transpurent, C= translucent, E = hazy*

**Table 8**

| Viscosity of Elastomer Blends as a Function of the Crosslinker and Volume Percent OMC | | | | |
| --- | --- | --- | --- | --- |
| Crosslinker | Paste Viscosity (*10^3cP) by Helipath Viscometer: 2.5rpm, Spindle T-D | | | |
| | 0% OMC | 6% OMC | 12% OMC | 18% OMC |
| Hexadiene | 367 | 339 | 242 | 70 |
| 7 DP PPG | 464 | 493 | 521 | 512 |
| 17 DP PPG | 546 | 663 | 762 | >800 |
| 34 DP PPG | 430 | 550 | 607 | 692 |

Example 5

*Compatibility of Gel Pastes containing a Polvalhyloxylene Crosslinked Silicone organic Elastomer with various personal ingredients*

Table 9 summarizes compatibility data for a silicone-organic elastomer gel paste based on a polypropylene glycol crosslinker formulated with a variety of personal care ingredients at a 15 wt % addition level vs. a silicone organic elastomer gel paste based on hexadiene crosslinker. Compatibility was based on assessing both appearance (clarity and homogeneity) and thickening behaviors. Table 10 shows similar compatibility tests but using 25 wt % actives. As summarized in these tables, compatibility improved by incorporating polypropylene glycol into the elastomer.

Table 9
Compatibility of Elastomer Gel Pastes in Isododecane Swelling Agent with 15 wt% Organic Actives

| Cross-linker | Crosslinker as % of Elastomer | Octyl Methoxy-cinnamate | Ethanol | C12-15 Alkyl Benzoate | Caprylic/ Capric Triglyceride | Sunflower Oil | Octyl Salicylate | Squalane | PPG-15 Stearyl Ether |
|---|---|---|---|---|---|---|---|---|---|
| Hexadiene | -3.6 | C | B | B | B | D | B | B | D |
| PPG | 20.5 | A | A | A | A | B | A | A | A |

5 A = highly compatible, B = slight incompatibility, C = marginal compatibility, D = incompatible. Rating based on observed clarity and thickening behaviors.

**Table 10**
**Compatibility of Elastomer Gel Pastes in Isododecane Swelling Agent with 25% Organic Actives**

| Crosslinker | Crosslinker as % of Elastomer | Octyl Methoxy-cinnamate | Ethanol | C12-15 Alkyl Benzoate | Caprylic/ Capric Triglyceride | Sunflower Oil | Octyl Salicylate | Squalane | PPG-15 Stearyl Ether |
|---|---|---|---|---|---|---|---|---|---|
| Hexadiene | -3.6 | D | D | C | D | D | B | D | D |
| PPG | 20.5 | B | B | A | A | D | A | D | D |

10 A = highly compatible, B = slight incompatibility, C = marginal compatibility, D = incompatible. Rating based on observed clarity and thickening behaviors.

Example 6

*Sensory Behavior of Gels containing a Polvalhyloxylene Crosslinked Silicone organic elastomer*

The sensory aesthetics of neat elastomer gel pastes were evaluated using a trained expert sensory panel. The resulting sensory data is shown in Charts 1 and 2, which compares the sensory aesthetics of a polyalkyloxylene crosslinked silicone organic elastomer paste (paste 2A) vs. a hexadiene crosslinked silicone organic elastomer paste. The results show the sensory profiles are similar.

**Sensory Behavior of Elastomer Blend Pastes in Isododecane: Comparison of Materials Crosslinked with Hexadiene versus Bis(allyl) Poly(propylene glycol)**

Chart 1

Chart 2

Example 7

*Reparation of a Gel containing Polyalkyloxylene Crosslinked Silicone organic elastomer having pendent polyoxyethylene groups*

Organohydrogensiloxane 2, monoallyl polyether groups [either poly(propylene oxide) or poly(ethylene oxide), polyalkyoxylene 4 or 5] and isododecane were charged into the reaction vessel and heated to 70°C. While stirring, 0.7g of a solution of 4000 catalyst (Karstedt's catalyst Platinum (0) complexed with 1,3-divinyl-1,1,3,3-tetramethyldisiloxane) was added. The reaction mixture was stirred at 70°C for 15 minutes. A polyalkyloxylene crosslinker (#2) was then added to the reaction mixture. The final reaction mixture was held at 70°C for three hours during which time a gel was formed. Actual gel formulations are shown in Tables 11 and 12. The gels were made into pastes using shear and additional solvent as shown in Table 13.

Table 11

| Formulation for bisallyl polyether crosslinked gel with poly(propylene oxide) pendent groups | | |
|---|---|---|
| Combine these three materials and heat to 70 °C in a capped glass jar | 25.9 g | Organohydrogensiloxane 2 |
| | 10.1 g | Polyalkyloxylene 4 |
| | 185.6 g | isododecane |
| While stirring, add platinum catalyst | 0.7 g | 4000 Catalyst (Pt) |
| 15min after catalyst addition, add these two materials while stirring | 20.2 g | Polyalkyloxylene 2 |
| | 13.2 g | isododecane |
| Hold reaction mixture at 70 °C for 3 hr. | | |

Table 12

| Formulation for bisallyl polyether crosslinked gel with poly(ethylene oxide) pendent groups | | |
|---|---|---|
| Combine these three materials and heat to 70 °C in a capped glass jar | 29.1 g | Organohydrogensiloxane 2 |
| | 4.4 g | Polyalkyloxylene 5 |
| | 185.6 g | isododecane |
| While stirring, add platinum catalyst | 0.7 g | 4000 Catalyst (Pt) |
| 15min after catalyst addition, add these two materials while stirring | 22.6 g | Polyalkyloxylene 2 |
| | 13.2 g | isododecane |
| Hold reaction mixture at 70 °C for 3 hr. | | |

Table 13

| Formulation for silicone organic elastomer pastes processed from gels in Tables 11 and 12 | | |
|---|---|---|
| shear step 1 | 56.9 g | silicone/polyether elastomer gel |
| shear step 2 | 26.2 g | isododecane |
| | 0.5 g | organovinylsiloxane |

The pastes were mixed with water (2 parts paste, 1 part water). The paste with poly(ethylene oxide) pendent groups formed a water-in-oil emulsion, while the paste with poly(propylene oxide) pendent groups did not form an emulsion.

Example 8

*Personal care formulations containing silicone-organic gels*

Personal care formulations containing representative silicone-organic gels of the present disclosure were prepared, as described in this example. The silicone-organic pastes used in these formulations were Paste 2A or 2B from Table 6. (that is, a formulation was made using each silicone organic gel, unless noted otherwise)

## Castor oil-based Lipstick Formulation

| Raw Material | INCI Name | Wt% |
|---|---|---|
| **Phase A** | | |
| castor oil | | 43.7 |
| Softisan 100 | Hydrogenated coco-glycerides | 8 |
| cerilla G | Candelilla Cera | 9 |
| Softisan 645 | Bis-Diglyderyl Polyacyladipate | 8 |
| Cerabeil Blanchie DAB | Cera alba | 3 |
| Cerauba T1 | Cera Carnauba | 2 |
| Trivent OC-G | Ticaprylin | 15 |
| Vitamin E acetate | Tocopheryl acetate | 0.5 |
| Propyl Paraben | | 0.1 |
| BHT(2,6-di-tert-butyl-4-methylphenol) | | 0.05 |
| Elastomer Blend | Silicone Organic paste 2A or 2B | 5 |
| Total phase A | | **94.3** |
| **Phase B** | | |
| COD 8008 | White | 1 |
| COD 8005 | Yellow | 3 |
| COD 8006 | Red | 1.7 |
| Total Phase B | | **5.7** |

Procedure:

1. Heat Phase A to 85° C.
2. Add Phase B.
3. Pour into lipstick molds.
4. Place in freezer for 60 min.
5. Remove from molds.

## Cyclopentasiloxane-based Lipstick Formulation with 5% Elastomer Blend

| Raw Material | INCI Name | % |
|---|---|---|
| **Phase A** | | |
| White ozokerite wax | | 4 |
| Cerilla G | Candellila wax | 11 |
| Eutanol G | Octyl dodecanol | 25 |
| Dow Corning 245 | Cyclopentasiloxane | 5 |
| Elastomer Blend | Silicone Organic Paste 2A or 2B | 5 |
| Petrolatum | Vaselinum | 4 |
| Fluilan | lanolin oil | 9 |
| Avocado oil | | 2 |
| Novol | Oleyl alcohol | 8 |
| pigment blend | | 27 |
| | | **100** |
| **Pigment blend** | | |
| Covasil TiO2 | | 5 |
| Dow Corning 245 | Cyclopentasiloxane | 77.5 |
| Covasil red W3801 | | 17.5 |
| | | **100** |

Procedure:

1. Heat phase A to 85°C except pigment blend.
2. Add pigment blend.
3. Pour formulation into lipstick mold.
4. Place in freezer for 60 min.
5. Remove from molds

## Liptissime Duo Stick

| Raw Material | | % |
|---|---|---|
| *Phase A* | | |
| Unipure Red LC 304 AS | CI 15850 and Triethoxycaprylylsilane | 6.4 |
| Unipure Black LC 989 AS-EM | CI 77499 and Triethoxycaprylylsilane | 0.8 |
| Dow Corning® PH-1555 HRI COSMETIC FLUID | Trimethyl Pentaphenyl Trisiloxane | 14.5 |
| | | |
| *Phase B* | | |
| Dow Corning® AMS-C30 COSMETIC WAX | C30-45 Alkyl Methicone (and) C30-45 Olefin | 6.8 |
| Dow Corning® 2503 COSMETIC WAX | Stearyl Dimethicone | 2.2 |
| Covalip LL 48 | Ozokerite and Euphorbia Cerifera (Candelilla) Wax and Isostearyl Alcohol and Isopropyl Palmitate and Myristyl Lactate and Synthetic Beeswax and Copernicia Cerifera (Carnauba) Wax and Quaternium-18 Hectorite and Propylene Carbonate and Ethylene/VA Copolymer and Propylparaben | 11.5 |
| Covasterol | Glyceryl Isostearate and Isostearyl Alcohol and Beta-Sitosterol and Butyrospermum Parkii (Shea Butter) and Euphorbia Cerifera (Candelilla) Wax | 0.5 |
| | | |
| *Phase C* | | |
| Covapearl Satin 931 AS | Mica and CI 77891 and Triethoxycaprylylsilane | 10 |
| Covafluid FS | Sodium Stearyl Fumarate | 1 |
| | | |
| *Phase D* | | |
| Dow Corning® 245 FLUID | Cyclopentasiloxane | 18 |
| Elastomer Blend | Silicone Organic Paste 2A or 2B | 27.8 |

Procedure:

1. Grind pigment of phase A in silicone with high shear mixer
2. Mix ingredients of phase B and heat to 80°C
3. Add phase A to phase B while stirring and continued heating
4. Add phase C with mixing
5. Add phase D under stirring and maintain the temperature at 70°C
6. Pour in the mould at 70°C

## Liquid Lipstick : Long lasting

| Raw Material | | % |
|---|---|---|
| | | |
| **Phase A** | | |
| Unipure Red LC 304 AS / LCW Sensient | | 3.6 |
| Unipure Red LC 3075 AS / LCW Sensient | | 3.6 |
| Isododecane | | 10 |
| | | |
| **Phase B** | | |
| Dow Corning® AMS-C30 COSMETIC WAX | | 6 |
| Isododecane | | 42.2 |
| | | |
| **Phase C** | | |
| Elastomer Blend | Silicone Organic Paste 2A or 2B | 26.4 |
| | | |
| **Phase D** | | |
| Paragon MEPB | | 0.2 |
| | | |
| **Phase E** | | |
| Covapearl Rich Gold 230 AS / LCW Sensient | | 8 |

Procedure:

1. Mix phase A ingredients together
2. Homogenize using a high shear mixer (Ultraturrax or Silverson type)
3. Heat Ingredient 4 to 80°C
4. Tare the beaker (final beaker), warm the isododecane up to 40°C covering the beaker with an aluminium foil
5. Add ingredient 4 to 5, stop heating and ensure a homogeneous mixture
6. Add phase A to phase B with gentle mixing
7. Add phase C with mixing
8. Add phase D with slow mixing
9. Add phase E with slow mixing
10. Finally compensate the solvent loss with isododecane

## Foundation Cream with elastomer blend

| Raw Material | | % |
|---|---|---|
| | | |
| **Phase A** | | |
| DC 2-1184 | | 11.0 % |
| TiO2 W877 | Titanium dioxide | 11.0 % |
| Yellow W 1802 | Iron Oxide | 2.5 % |
| Red W 3801 | Iron Oxide | 1.5 % |
| Black W 9801 | Iron Oxide | 0.6 % |
| **Phase B** | | |
| Elastomer Blend | Silicone Organic Paste 2A or 2B | 12.0 % |
| Sepicide HB | Parabens blend | 0.5 % |
| Dow Corning® 5200 | Cyclomethicone (and) PEG/PPG-18/18 Dimethicone | 2.0 % |
| **Phase C** | | |
| Tween 20 | Polysorbate 20 | 0.5 % |
| NaCl | | 1.0 % |
| Distilled water | | 57.4 % |

Procedure:

1. Mix ingredients of Phase A and homogenize using a high shear mixer
2. Add Elastomer Blend, when melted, add remain of Phase B
3. Mix ingredients of Phase C in another beaker
4. Add Phase C very slowly into Phase A+B under agitation (1200 rpm)
5. When addition is completed, leave under agitation for an additional 5 minutes and pass through a homogenizer

**Tinted sunscreen**

| Phase A | | |
|---|---|---|
| Dow Corning® 5200 FORMULATION AID | | 2.0 |
| Elastomer Blend | Silicone Organic Paste 2A | 20.0 |
| Dow Corning® 556 FLUID | | 3.0 |
| Parsol MCX | Octyl Methoxycinnamate | 6.0 |
| Phase B | | |
| Pigment blend | | 20 |
| Phase C | | |
| Deionized water | | 47.4 |
| Disodium EDTA | | 0.2 |
| Sodium Chloride | | 1 |
| Nipaguard DMDH | | 0.2 |
| Polysorbate 20 | Tween 20 | 0.2 |
| | | |
| | Pigment blend | |
| 2-1184 | | |
| | | |

Procedure:

1. Mix phase A ingredients together
2. Grind Phase B ingredients together and add to phase A with mixing
3. Mix phase C ingredients together
4. Check pH of water phase (5.5-6.5) and correct if necessary
5. Add phase C to AB blend, slowly and with turbulent mixing (about 1000 rpm)
6. Continue mixing for 10 minutes at the same speed
7. Pass the mixture through high shear device to get uniform particle size distribution

## Shower Gel with Smooth after Feel

| Raw Material | Commercial name | % |
|---|---|---|
| **Phase A** | | |
| Distilled Water | | 57.37 |
| Crothix 2602 | | 0.5 |
| Propylene Gycol | | 1.0 |
| **Phase B** | | |
| Sodium Laureth Sulfate | Empicol ESB 3 | 20 |
| Ammonium Laureth Sulfate | Empicol EAC 70 | 6.43 |
| Cocamidopropyl betaine | Amonyl 380 BA | 8.0 |
| Cocamide MIPA | Ninol M-10 | 4.0 |
| Elastomer Blend | Silicone Organic Paste 2A | 2.0 |
| **Phase C** | | |
| Nipaguard DMDH | | 0.2 |
| **Phase D** | | |
| Citric acid 50% | | 0.5 |

Procedure:

1. Mix ingredients of Phase A
2. Mix Phase B ingredients together and add to Phase A with mixing
3. Add Phase C and mix
4. Ajust the pH with phase D at 5.5 - 6
5. Pour into containers

The present invention relates to following Aspects:

1. A personal or healthcare composition comprising a silicone organic elastomer gel derived from;

i) a silicone organic elastomer comprising a reaction product of;

A) an organohydrogensiloxane comprising siloxy units of average formula

$$(R_13SiO_{0.5})v(R_22SiO)x(R_2HSiO)y$$

wherein $R^1$ is hydrogen or $R^2$,
$R^2$ is a monovalent hydrocarbyl,

$v \geq 2$, $x \geq 0$, $y \geq 2$,
B) a polyoxyalkylene comprising the average formula

$$R^3O-[(C_2H_4O)_c(C_3H_6O)_d(C_4H_8O)_e]-R^3$$

wherein

$R^3$ is a monovalent unsaturated aliphatic hydrocarbon group containing 2 to 12 carbon atoms,
c is from 0 to 100,

d is from 0 to 100,
e is from 0 to 100,

with a proviso the ratio of (d + e)/(c + d + e) is greater than 0.5,
C) a hydrosilylation catalyst, and
D) an optional organic compound having one terminal unsaturated aliphatic hydrocarbon group,

ii) a carrier fluid, and

E) a personal or healthcare active.

2. The composition of aspect 1 wherein the silicone organic elastomer gel contains

2 - 95 weight % of i) the silicone organic elastomer, and
5 - 98 weight % of ii) the carrier fluid.

3. The composition of aspect 1 or 2 wherein the carrier fluid is an organic liquid.

4. The composition of aspect 3 wherein the organic liquid is an aliphatic hydrocarbon.

5. The composition of aspect 1 wherein the molar ratio of the unsaturated aliphatic hydrocarbon groups in B) to the SiH units in A) is greater than 1.

6. The composition of aspect 1 wherein A) the organohydrogensiloxane is selected from an organohydrogensiloxane having the average formula

$$(R^1_3SiO_{0.5})_v(R^2_2SiO)_x(R^2HSiO)_y(R^2SiO_{1.5})_z,$$

$$(R^1_3SiO_{0.5})_v(R^2_2SiO)_x(R^2HSiO)_y(SiO_2)_w,$$

$$(R^1_3SiO_{0.5})_v(R^2_2SiO)_x(R^2HSiO)_y(SiO_2)_w(R^2SiO_{1.5})_z$$

or any mixture thereof,
where

$R^1$ is hydrogen or $R^2$,
$R^2$ is a monovalent hydrocarbyl,
and $v \geq 2$, $w \geq 0$, $x \geq 0$, $y \geq 2$, and z is $\geq 0$.

7. The composition of aspect 1 wherein the organohydrogensiloxane is a dimethyl, methyl-hydrogen polysiloxane having the average formula;

$$(CH_3)_3SiO[(CH_3)_2SiO]_x[(CH_3)HSiO]_ySi(CH_3)_3$$

where $x \geq 0$, and $y \geq 2$.

8. The composition of aspect 1 wherein the polyoxyalkylene is selected from

$$H_2C=CHCH_2O[C_3H_6O]_dCH_2CH=CH_2$$

$$H_2C=C(CH_3)CH_2O[C_3H_6O]_dCH_2C(CH_3)=CH_2$$

$$HC=CCH_2O[C_3H_6O]_dCH_2C\equiv CH, or$$

$$HC\equiv CC(CH_3)_2O[C_3H_6O]_dC(CH_3)_2C\equiv CH$$

where d is 1 to 100.

9. The composition of aspect 1 wherein the hydrosilylation catalyst is a platinum group metal-containing catalyst.

10. The composition of aspect 1 wherein the organohydrogensiloxane is a dimethyl, methyl-hydrogen polysiloxane having the average formula;

$$(CH_3)_3SiO[(CH_3)_2SiO]_x[(CH_3)HSiO]_ySi(CH_3)_3$$

where $x \geq 0$, and $y \geq 2$,
the polyoxyalkylene is $H_2C=CHCH_2O[C_3H_6O]_dCH_2CH=CH_2$ where d is 1 to 100, and the carrier fluid is an aliphatic hydrocarbon.

11. The composition of aspect 10 wherein the aliphatic hydrocarbon is isododecane.

12. The composition of aspect 1 wherein E) is a personal care active selected from a vitamin, sunscreen, plant extract, or fragrance.

13. The composition of aspect 1 wherein E) is a health care active selected from a topical drug active, protein, enzyme, antifugual, or antimicrobial agent.

14. The composition of aspect 1 wherein component E) is octyl methoxycinnamate.

15. The composition of aspect 1 wherein the personal care composition is selected from a color cosmetic, a lipstick, a foundation, a shampoo, a hair conditioner, a hair fixative, a shower gel, a skin moisturizer, a skin conditioner, a body conditioner, a sun protection product, an antiperspirant, and a deodorant.

## Claims

1.  A personal or healthcare composition comprising a silicone organic elastomer gel derived from;

    i) a silicone organic elastomer comprising a reaction product of;

    A) a linear or branched organohydrogensiloxane comprising siloxy units of average formula

    $$(R^1_3SiO_{0.})_v(R^2_2SiO)_x(R^2HSiO)_y$$

    wherein $R^1$ is hydrogen or $R^2$,
    $R^2$ is a monovalent hydrocarbyl,
    $v \geq 2$, $x \geq 0$, $y \geq 2$,

    B) a polyoxyalkylene comprising the average formula

    $$R^3O-[(C_2H_4O)_c(C_3H_6O)_d(C_4H_8O)_e]-R^3$$

    wherein

    $R^3$ is a monovalent unsaturated aliphatic hydrocarbon group containing 2 to 12 carbon atoms,
    c is from 0 to 100,
    d is from 0 to 100,
    e is from 0 to 100,

    with a proviso the ratio of (d + e)/(c + d + e) is greater than 0.5,
    C) a hydrosilylation catalyst, and
    D) an optional organic compound having one terminal unsaturated aliphatic hydrocarbon group,

    ii) a carrier fluid, and

E) a personal active selected from a vitamin, plant extract, or fragrance; or a healthcare active selected from a topical drug active, protein, enzyme, antifungal, or antimicrobial agent.

2. The composition of claim 1 wherein the silicone organic elastomer gel contains

2 - 95 weight % of i) the silicone organic elastomer, and
5 - 98 weight % of ii) the carrier fluid.

3. The composition of claim 1 or 2 wherein the carrier fluid is an organic liquid.

4. The composition of claim 3 wherein the organic liquid is an aliphatic hydrocarbon.

5. The composition of claim 1 wherein the molar ratio of the unsaturated aliphatic hydrocarbon groups in B) to the SiH units in A) is greater than 1.

6. The composition of claim 1 wherein A) the linear or branched organohydrogensiloxane is selected from a linear or branched organohydrogensiloxane having the average formula

$$(R^1_3SiO_{0.5})_v(R^2_2SiO)_x(R^2HSiO)_y(R^2SiO_{1.5})_z,$$

$$(R^1_3SiO_{0.5})_v(R^2_2SiO)_x(R^2HSiO)_y(SiO_2)_w,$$

$$(R^1_3SiO_{0.5})_v(R^2_2SiO)_x(R^2HSiO)_y(SiO_2)_w(R^2SiO_{1.5})_z$$

or any mixture thereof,
where

$R^1$ is hydrogen or $R^2$,
$R^2$ is a monovalent hydrocarbyl,
and $v \geq 2$, $w \geq 0$, $x \geq 0$, $y \geq 2$, and $z$ is $\geq 0$.

7. The composition of claim 1 wherein the linear or branched organohydrogensiloxane is a dimethyl, methyl-hydrogen polysiloxane having the average formula;

$$(CH_3)_3SiO[(CH_3)_2SiO]_x[(CH_3)HSiO]_ySi(CH_3)_3$$

where $x \geq 0$, and $y \geq 2$.

8. The composition of claim 1 wherein the polyoxyalkylene is selected from

$$H_2C=CHCH_2O[C_3H_6O]_dCH_2CH=CH_2$$

$$H_2C=C(CH_3)CH_2O[C_3H_6O]_dCH_2C(CH_3)=CH_2$$

$$HC\equiv CCH_2O[C_3H_6O]_dCH_2C\equiv CH, \text{ or}$$

$$HC\equiv CC(CH_3)_2O[C_3H_6O]_dC(CH_3)_2C\equiv CH$$

where d is 1 to 100.

9. The composition of claim 1 wherein the hydrosilylation catalyst is a platinum group metal-containing catalyst.

10. The composition of claim 1 wherein the linear or branched organohydrogensiloxane is a dimethyl, methyl-hydrogen polysiloxane having the average formula;

$$(CH_3)_3SiO[(CH_3)_2SiO]_x[(CH_3)HSiO]_ySi(CH_3)_3$$

where $x \geq 0$, and $y \geq 2$,

the polyoxyalkylene is $H_2C=CHCH_2O[C_3H_6O]_dCH_2CH=CH_2$ where d is 1 to 100, and the carrier fluid is an aliphatic hydrocarbon.

11. The composition of claim 10 wherein the aliphatic hydrocarbon is isododecane.

12. The composition of claim 1 wherein the personal care composition is selected from a color cosmetic, a lipstick, a foundation, a shampoo, a hair conditioner, a hair fixative, a shower gel, a skin moisturizer, a skin conditioner, a body conditioner, an antiperspirant, and a deodorant.

Europäisches
Patentamt

European
Patent Office

Office européen
des brevets

# EUROPEAN SEARCH REPORT

Application Number

EP 16 17 1079

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 412 004 A (TACHIBANA KIYOMI [JP] ET AL) 2 May 1995 (1995-05-02) * examples 5,11,14 * * page 8, line 12 - page 9, line 33 * ----- | 1-12 | INV. A61K8/04 A61K8/894 A61K8/31 A61K8/37 A61Q1/06 A61Q1/02 A61Q17/04 A61Q19/10 A61Q5/06 A61Q5/12 A61Q5/00 A61Q19/00 A61Q5/02 A61Q15/00 |
| X | EP 1 148 099 A (DOW CORNING TORAY SILICONE [JP]) 24 October 2001 (2001-10-24) * paragraphs [0001], [0004], [0005], [0007] - [0009], [0012], [0013]; examples 1-13 * ----- | 1-12 | |
| X | DOW CORNING: "Product Information: Dow Corning 9011 . SILICONE ELASTOMER BLEND", INTERNET CITATION , XP002330255, Retrieved from the Internet: URL:http://www.dowcorning.com/DataFiles/090007b580108c93.pdf [retrieved on 2005-06-01] | 1-12 | |
| Y | * the whole document * ----- | 1-12 | TECHNICAL FIELDS SEARCHED (IPC) |
| Y | WO 2004/100906 A (PROCTER & GAMBLE [US]; CLAPP MANNIE LEE [US]; TAYLOR REBECCA ANN [US];) 25 November 2004 (2004-11-25) * page 4, line 21 - page 5, line 20 * * page 6, line 14 - line 18 * * examples 5,11 * ----- | 1-12 | A61Q A61K |
| E | WO 2009/006091 A (DOW CORNING [US]; KENNAN JOHN JOSEPH [US]; MESSNER KATHRYN ELIZABETH []) 8 January 2009 (2009-01-08) * paragraphs [0007] - [0041], [0044] - [0045]; examples * * tables 14-15 * ----- -/-- | 1-12 | |

The present search report has been drawn up for all claims

| Place of search | Date of completion of the search | Examiner |
|---|---|---|
| Munich | 2 August 2016 | Krattinger, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 16 17 1079

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WO 2007/109240 A (DOW CORNING [US]; LIN SHAOW [US]) 27 September 2007 (2007-09-27) * paragraphs [0006] - [0021], [0031], [0037] - [0060], [0063]; examples 3-22 * * page 29, line 40 * | 1-12 | |

TECHNICAL FIELDS
SEARCHED (IPC)

The present search report has been drawn up for all claims

| Place of search | Date of completion of the search | Examiner |
|---|---|---|
| Munich | 2 August 2016 | Krattinger, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 17 1079

This annex lists the patent family members relating to the patent documents cited in the above-mentioned European search report.
The members are as contained in the European Patent Office EDP file on
The European Patent Office is in no way liable for these particulars which are merely given for the purpose of information.

02-08-2016

| Patent document cited in search report | | Publication date | Patent family member(s) | | Publication date |
|---|---|---|---|---|---|
| US 5412004 | A | 02-05-1995 | EP | 0545002 A1 | 09-06-1993 |
| | | | US | 5412004 A | 02-05-1995 |
| EP 1148099 | A | 24-10-2001 | EP | 1148099 A2 | 24-10-2001 |
| | | | JP | 4783491 B2 | 28-09-2011 |
| | | | JP | 2001294754 A | 23-10-2001 |
| | | | US | 2001041771 A1 | 15-11-2001 |
| WO 2004100906 | A | 25-11-2004 | AU | 2004238310 A1 | 25-11-2004 |
| | | | CA | 2524306 A1 | 25-11-2004 |
| | | | EP | 1622578 A2 | 08-02-2006 |
| | | | KR | 20060009904 A | 01-02-2006 |
| | | | MX | PA05011947 A | 02-02-2006 |
| | | | WO | 2004100906 A2 | 25-11-2004 |
| WO 2009006091 | A | 08-01-2009 | AT | 529090 T | 15-11-2011 |
| | | | CN | 101686901 A | 31-03-2010 |
| | | | EP | 2167014 A2 | 31-03-2010 |
| | | | JP | 5441899 B2 | 12-03-2014 |
| | | | JP | 5841103 B2 | 13-01-2016 |
| | | | JP | 2010532413 A | 07-10-2010 |
| | | | JP | 2013224447 A | 31-10-2013 |
| | | | KR | 20100053528 A | 20-05-2010 |
| | | | US | 2010330011 A1 | 30-12-2010 |
| | | | WO | 2009006091 A2 | 08-01-2009 |
| WO 2007109240 | A | 27-09-2007 | EP | 2001933 A2 | 17-12-2008 |
| | | | JP | 5424866 B2 | 26-02-2014 |
| | | | JP | 2009530476 A | 27-08-2009 |
| | | | KR | 20080108558 A | 15-12-2008 |
| | | | US | 2010158824 A1 | 24-06-2010 |
| | | | WO | 2007109240 A2 | 27-09-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 60975318 B
- US 5880210 A
- US 5760116 A
- US 5811487 A
- US 6200581 B
- US 5236986 A
- US 6331604 B
- US 6262170 B
- US 6531540 B
- US 6365670 B
- US 3419593 A, Willing
- US 5175325 A, Brown
- US 3989668 A, Lee
- US 5036117 A, Chang
- US 3159601 A, Ashby
- US 3220972 A, Lamoreaux
- US 3296291 A, Chalk
- US 3516946 A, Modic
- US 3814730 A, Karstedt
- US 3928629 A, Chandra
- US 01722901 A
- US 6987157 B
- US 6013682 A

- WO 03101412 A2
- US 4122029 A, Gee
- US 5387417 A, Rentsch
- US 6051216 A
- US 5919441 A
- US 5981680 A
- WO 2004060271 A
- WO 2004060101 A
- WO 2004060276 A
- WO 03105801 A
- US 20030235553 A
- US 20030072730 A
- US 20030170188 A
- EP 1266647 A
- EP 1266648 A
- EP 1266653 A
- WO 03105789 A
- WO 2004000247 A
- WO 03106614 A
- WO 2004054523 A
- US 20040180032 A
- WO 2004054524 A

**Non-patent literature cited in the description**

- Code of Federal Regulations. United States Department of Health & Human Services Food and Drug Administration

- **S. ARCTANDER.** Perfume and Flavour Chemicals. 1969